# EUROPEAN PATENT APPLICATION

(11) **EP 4 516 910 A1**
(43) Date of publication of application: **05.03.2025**
(21) Application number: 23795688.3
(22) Date of filing: 20.06.2023
(51) Int. Cl.: C12N 15/62, C12N 9/22, C07K 14/195

(54) **ISOLATED CAS13 PROTEINS, GENE EDITING SYSTEM BASED THEREON, AND USE THEREOF**

(30) Priority: 26.04.2022 CN 202210446714; 24.04.2023 CN 202310447170
(71) Applicant: Beijing Institute for Stem Cell and Regenerative Medicine, Beijing 100101 (CN); Institute Of Zoology, Chinese Academy Of Sciences, Beijing 100101 (CN)
(72) Inventor: LI, Wei, Beijing 100101 (CN); ZHOU, Qi, Beijing 100101 (CN); HU, Yanping, Beijing 100101 (CN); CHEN, Yangcan, Beijing 100101 (CN)
(74) Representative: Lavoix
(86) International application number: PCT/CN2023/101348
(87) International publication number: WO 2023/208256

(57) **Abstract**

The present application relates to isolated novel CRISPR/Cas13 proteins, a gene editing systems based thereon, and a method for using said proteins for RNA level gene editing. Provided are non-naturally occurring or engineered RNA targeting systems, and said systems each have a novel Cas13 effector protein that targets RNA and at least one type of guide molecule.

## Description

### TECHNICAL FIELD

The present application is in the field of biotechnology and specifically relates to an isolated Cas13 protein, a gene editing system based on it and a method of using them for RNA level gene editing.

### TECHNICAL BACKGROUND

Gene editing technology makes it possible to modify DNA sequence anchor points, such as the first-generation gene editing tools, zinc finger nucleases(ZFNs), and the second-generation gene editing tools, transcription activator-like effector nucleases(TALENs), the third generation gene editing tools, type II and V clustered regularly interspaced short palindromic repeats (CRISPR)/Cas (CRISPR-associated proteins) can be used for targeted genome modification, but these gene editing systems can only target the genome and foreign DNA, but cannot perform targeted editing on RNA in vivo.

Type VI CRISPR/Cas13 system is the natural immune system from archaea and bacteria, which is different from previous gene editing tools, it uses the principle of complementary nucleic acid base pairing to identify target RNA sequences and guide Cas effector proteins for targeted cleavage. It has strong applicability, simple design, and high efficiency.

Among them, type VI-D CRISPR/Cas13 gene editing system is the most widely used type VI CRISPR/Cas system. This system can perform targeted editing of single-stranded RNA in prokaryotes by recognizing and cutting the protospacer flanking site (PFS) sequence on both sides of the targeted polynucleotide. And in eukaryotes, the VI-D type CRISPR/Cas system has no PFS sequence for targeted editing of RNA. At the same time, compared with other type VI systems, the type VI-D system has a smaller protein size and has better prospects in gene therapy based on adeno-associated virus (AAV). In the huge and diverse metagenomes, there are hidden microorganisms that have not been cultured or even discovered. There may be a large number of undiscovered type VI CRISPR/Cas13 systems. Finding and cloning Cas proteins with better characteristics among these microorganisms is what people expect.

### SUMMARY OF THE INVENTION

The purpose of this application is to find novel Cas13 gene editing systems in metagenomes and develop their uses. Specifically, provided is that:
**1.** Anisolated Cas13 nuclease protein, the amino acid sequence of the Cas13 protein comprises:
   a) the amino acid sequence shown in any one of SEQ ID NOs. 1 to 28; or
   b) an amino acid sequence having at least 80% sequence identity with any one of SEQ ID NOs. 1 to 28 and having RNA cleavage activity.
**2.** The Cas13 nuclease protein of item 1, wherein the Cas13 nuclease protein is Cas13bt1, Cas13bt2, Cas13g, Cas13h, Cas13i, Cas13j or Cas13k protein.
**3.** An engineered Cas13 nuclease effector protein, comprising:
   a) the amino acid sequence shown in any one of SEQ ID NOs. 1 to 28; or,
   b) an amino acid sequence having at least 80% sequence identity with any one of SEQ ID NOs. 1 to 28 and having RNA cleavage activity.
**4.** The effector protein of item 3, wherein the Cas13 nuclease protein loses its catalytic activity through amino acid mutation, for example, the HEPN domain (RxxxxH motif) of the Cas13 nuclease protein at its C-terminal and/or N-terminal forms dCas13 protein through amino acid mutations.
**5.** The effector protein of item 3 or 4, further comprising a functional domain fused to the Cas13 nuclease protein.
**6.** The effector protein of item 5, wherein the functional domain is selected from one or more of the following: translation initiation domain, translation repression domain, transactivation domain, epigenetic modification domain, nucleobase editing domain, reverse transcriptase domain, reporter domain and nuclease domain.
**7.** The effector protein of item 6, wherein the nucleobase editing domain is adenosine deaminase, cytidine deaminase or their catalytic domains.
**8.** A polynucleotide, encoding the Cas13 nuclease protein of item 1 or 2 or the effector protein of any one of items 3-7.
**9.** A vector, comprising the polynucleotide of item 8, preferably the vector is a plasmid or a lentivirus.
**10.** An engineered CRISPR-Cas13 gene editing system, comprising:
   (a) the engineered Cas13 nuclease effector protein of any one of items 3-7, or the nucleic acid encoding the effector protein; and
   (b) crRNA, comprising a spacer sequence complementary to a target sequence, when using engineered Cas13 nuclease effector proteins as shown in SEQ ID NOs. 1-28, the crRNA also comprises direct repeat (DR) sequences that have at least 80% identity with the sequences shown in SEQ ID NOs. 29-56;
   wherein, the engineered Cas13 nuclease effector protein and the crRNA can form a CRISPR complex that specifically binds to a target nucleic acid comprising the target sequence and induces modification of the target nucleic acid.
**11.** A kit, comprising the engineered CRISPR-Cas13 gene editing system of item 10.
**12.** Use of the engineered CRISPR-Cas13 nuclease effector protein of any one of items 3-7, the engineered CRISPR-Cas13 gene editing system of item 10 in the preparation of a medicament for the treatment of diseases or disorders associated with nucleic acid mutations in cells of an individual.
**13.** A method for modifying a cell comprising a target nucleic acid, comprising contacting the cell with the engineered Cas13 nuclease effector protein of any one of items 3-7, the engineered CRISPR-Cas13 gene editing system of item 10, thereby achieving modification of the target nucleic acid in the cell.
**14.** Use of the engineered Cas13 nuclease effector protein of any one of items 3-7, the engineered CRISPR-Cas13 gene editing system of item 10 in the preparation of a medicament for the treatment of diseases or disorders associated with nucleic acid mutations in an individual.

This application also provides:
**1.** An isolated Cas13 nuclease protein, the amino acid sequence of the Cas13 nuclease protein is:
   a) the amino acid sequence shown in any one of SEQ ID NOs. 1 to 28; or
   b) an amino acid sequence having at least 80% sequence identity with any one of SEQ ID NOs. 1 to 28 and having RNA cleavage activity.
**2.** The Cas13 nuclease protein of item 1, wherein the Cas13 nuclease protein is Cas13bt1, Cas13bt2, Cas13g, Cas13h, Cas13i, Cas13j or Cas13k protein, preferably Cas13g3.
**3.** An engineered Cas13 nuclease effector protein, comprising a Cas13 nuclease protein, the Cas13 nuclease protein comprising:
   a) the amino acid sequence shown in any one of SEQ ID NOs. 1 to 28; or,
   b) an amino acid sequence having at least 80% sequence identity with any one of SEQ ID NOs. 1 to 28 and having RNA cleavage activity.
**4.** The effector protein of item 3, wherein the Cas13 nuclease protein loses its catalytic activity through amino acid mutation, for example, the HEPN domain (RxxxxH motif) of the Cas13 nuclease protein at its C-terminal and/or N-terminal forms dCas13 protein through amino acid mutations.
**5.** The effector protein of item 3 or 4, further comprising a functional domain fused to the Cas13 nuclease protein.
**6.** The effector protein of item 5, wherein the functional domain is selected from one or more of the following: translation initiation domain, translation repression domain, transactivation domain, epigenetic modification domain, nucleobase editing domain, reverse transcriptase domain, reporter domain and nuclease domain.
**7.** The effector protein of item 6, wherein the nucleobase editing domain is adenosine deaminase, cytidine deaminase or their catalytic domains.
**8.** A polynucleotide, encoding the Cas13 nuclease protein of item 1 or 2 or the effector protein of any one of items 3-7.
**9.** A vector, comprising the polynucleotide of item 8, wherein the vector is a plasmid or a virus, and the virus is preferably a lentivirus.
**10.** An engineered CRISPR-Cas13 gene editing system, comprising:
   (a) the nuclease of item 1 or 2, the engineered Cas13 nuclease effector protein of any one of items 3-7, or the nucleic acid encoding the effector protein; and
   (b) crRNA, comprising a spacer sequence complementary to a target sequence in a target nucleic acid;
   wherein, the engineered Cas13 nuclease effector protein and the crRNA can form a CRISPR complex that specifically binds to a target nucleic acid comprising the target sequence and induces modification of the target nucleic acid.
**11.** The engineered CRISPR-Cas13 gene editing system of item 10, wherein the crRNA further comprises a direct repeat (DR) sequence, preferably the direct repeat (DR) sequence comprises any one of SEQ ID NOs. 29 to 56. or a sequence having at least 80% identity with the sequence shown in any one of SEQ ID NOs. 29 to 56.
**12.** A kit, comprising the engineered CRISPR-Cas13 gene editing system of item 10 or 11.
**13.** Use of the Cas13 nuclease protein of item 1 or 2, the engineered CRISPR-Cas13 nuclease effector protein of any one of items 3-7, the engineered CRISPR-Cas13 gene editing system of item 10 or 11 in the preparation of a medicament for the treatment of diseases or conditions associated with nucleic acid mutations in cells of an individual.
**14.** A method for modifying a cell comprising a target nucleic acid, comprising contacting the cell with the Cas13 nuclease protein of item 1 or 2, the engineered Cas13 nuclease effector protein of any one of items 3-7, the engineered CRISPR-Cas13 gene editing system of item 10 or 11, thereby achieving modification of the target nucleic acid in the cell.
**15.** A composition comprising the Cas13 nuclease protein of item 1 or 2, the engineered Cas13 nuclease effector protein of any one of items 3-7, the engineered CRISPR-Cas13 gene editing system of item 10 or 11, for modification of nucleic acids.

### Beneficial technical effects achieved by the technical solution of this application

This application analyzed metagenomic data and finally identified 5 novel isoforms and a total of 75 Cas13 proteins. The novel Cas13 isoforms are named as Cas13g-k; wherein the Cas13g protein is approximately 800 amino acids in size, and the Cas13h-k protein is approximately 1100 amino acids in size. These novel proteins all demonstrate good targeted cleavage activity at the RNA level. We report that the newly discovered Cas13g3 protein can be engineered for programmable RNA editing. Cas13g3 has a size of 767aa and is considered to be the most efficient small Cas13 protein currently and can be used as a promising mammalian RNA editing tool.

### BRIEF DESCRIPTION OF THE DRAWINGS

Figure 1 shows the flow chart of identification of a total of 75 Cas13 proteins in 5 novel isoforms through analysis of metagenomic data.
Figure 2 shows a schematic diagram of the protein homology between the 7 isoforms and the 4 old isoforms and the average protein size of each isoform.
Figure 3 shows another schematic diagram of the protein homology between the 7 isoforms and the 4 old isoforms.
Figure 4 shows a schematic diagram of the spacer-directed repeat sequence of the Loci site of Cas13bt1-Cas13k.
Figure 5 shows a schematic diagram of the HEPN domain at the C-terminus and N-terminus of Cas13bt1-Cas13k.
Figure 6 shows the ratio of RxxxxH motifs of Cas13bt1-Cas13k.
Figure 7 is a schematic diagram showing the sequence conservation of the RxxxxH motif of the HEPN domain at the C-terminus and N-terminus of Cas13bt1-Cas13k.
Figure 8 shows a schematic diagram of the predicted structure of the DR sequence of Cas13bt1-Cas13k.
Figure 9 shows the distribution ratio of Cas13a-Cas13k in microorganisms in various environments.
Figure 10 shows the existing ratio of Cas13a-Cas13k in nature.
Figures 11A and 11B are schematic diagrams of the experimental process for analyzing the RNA cleavage activity of Cas13 protein in HEK293T cells.
Figure 12 is a comparison of the efficiency of knocking down mCherry mRNA using 3'-DR or 5'-DR-crRNA.
Figures 13A and 13B show the results of knocking down mCherry mRNA to screen Cas13 proteins with high editing efficiency.
Figures 14A and 14B are real time quantitative reverse transcription PCR (RT-qPCR) results of novel Cas13-mediated ANXA4 mRNA knockdown.
Figure 15 shows the quantitative RT-qPCR results of endogenous mRNA knockdown mediated by RfxCas13d, Cas13X1, Cas13bt1-11 and Cas13g3.
Figure 16 shows the RT-qPCR results of novel Cas13d-mediated knockdown of ANXA4 mRNA.
Figure 17 shows the RNA-seq analysis results of ANXA4 mRNA knockdown mediated by RfxCas13d, Cas13X1, Cas13bt1-11 and Cas13g3.
Figures 18A and 18B show the trans-activity assessment of RfxCas13d, Cas13X1, Cas13bt1-11 and Cas13g3.
Figures 19A and 19B show analysis of optimal spacer length for the Cas13g3 system.
Figure 20 shows Cas13g3 system PFS preference analysis.
Figure 21 shows the effect of NLS interference activity on the Cas13g3 system.
Figure 22 shows a comparison of the RNA interference capabilities of the Cas13g3 system and other Cas13 systems.

### DETAIL DESCRIPTION OF THE PRESENT INVENTION

Specific embodiments of the present invention will be described in more detail below with reference to the accompanying drawings. Although specific embodiments of the present invention are shown in the drawings, it should be understood that the present invention may be embodied in various forms and should not be limited to the embodiments set forth herein. Rather, these embodiments are provided to provide a thorough understanding of the present invention, and to fully convey the scope of the present invention to those skilled in the art.

It should be noted that certain words are used in the description and claims to refer to specific components. Those skilled in the art will understand that skilled persons may use different names to refer to the same component. This specification and the claims do not use difference in nouns as a way to distinguish components, but rather use differences in functions of the components as a criterion for distinction. If the words "comprise" or "include" mentioned throughout the specification and claims are open-ended terms, they should be interpreted as "include but not limited to." The following descriptions of the specification are preferred embodiments for implementing the present invention. However, the descriptions are for the purpose of general principles of the specification and are not intended to limit the scope of the present invention. The protection scope of the present invention shall be determined by the appended claims.

As used herein, "substantially free" with respect to a particular component is used herein to mean that the particular component is not purposefully formulated into the composition and/or is present only as a contaminant or in trace amounts. Therefore, the total amount of a particular component resulting from any accidental contamination of the composition is less than 0.05%, preferably less than 0.01%. Most preferred are compositions in which the specific component is present in an amount undetectable by standard analytical methods.

As used in this specification, "a" or "an" may mean one or more. As used in the claims, the word "a" or "an" when used with the word "comprising" can mean one or more than one.

The term "or" is used in the claims to mean "and/or" unless it is expressly stated that only alternatives are to be referred to or the alternatives are mutually exclusive, although this disclosure supports reference to only alternatives and "and/or" definition. As used herein, "another" may mean at least a second or more.

Throughout this application, the term "about" is used to indicate that a value includes the inherent variation in error of the device, the method used to determine the value, or the variation that exists between study subjects.

In a first aspect, the present application provides an isolated Cas13 nuclease protein.

In a specific embodiment, provided is an isolated Cas13 nuclease protein, and the amino acid sequence of the Cas13 protein comprises: a) the amino acid sequence shown in any one of SEQ ID NO. 1 to 28; or, b) an amino acid sequence having at least 80% sequence identity with any one of SEQ ID NOs. 1 to 28 and having RNA cleavage activity. In yet another specific embodiment, provided is a Cas13 nuclease protein, wherein the Cas13 protein is Cas13bt1, Cas13bt2, Cas13g, Cas13h, Cas13i, Cas13j or Cas13k protein.

In the context of this specification, the terms Cas13a and C2c2 are used interchangeably. It was originally discovered by researchers at the Broad Institute. In 2015, Shmakov and colleagues used Cas1 as a "bait" to identify CRISPR-associated proteins in bacterial genomes. Through this analysis, they identified 53 candidate genes, divided into three major categories: C2c1, C2c2 and C2c3. C2c1 and C2c3 are somewhat similar to Cpf1, except that they require both tracrRNA and crRNA to cleave DNA targets, whereas Cpf1 only requires crRNA. The difference between C2c2 (that is, Cas13a) and Cas9 is that Cas13a binds and cuts RNA, while Cas9 cuts DNA. Cas9 utilizes tracrRNA and crRNA to bind and cleave DNA targets. Cas13a only requires a 24-base crRNA, which interacts with the Cas13a molecule through a uracil-rich stem-loop structure and promotes target cleavage through a series of conformational changes of Cas13a. Like Cas9, Cas13a can also tolerate a single mismatch between crRNA and the target sequence, but if there are two mismatches, the cutting efficiency is greatly reduced. Its PFS sequence (equivalent to PAM sequence) is located at the 3' end of the spacer region and consists of A, U or C bases. Another special feature of Cas13a is that once Cas13a recognizes and cleaves the RNA target specified by the crRNA sequence, it enters an enzymatic "activation" state, at which time it will bind and cleave other RNAs regardless of whether they are homologous to crRNA, or whether there is a PFS; this is very different from Cas9. In the Cas13 system, tracrRNA is not required, only crRNA is required; and crRNA is composed of a DR sequence (that is, a direct repeat sequence) and a spacer sequence (a spacer sequence, that is, a nucleotide sequence complementary to the target sequence).

In the second aspect, the present application provides an engineered Cas13 nuclease effector protein

In a specific embodiment, provided is an engineered Cas13 nuclease effector protein, comprising: a) an amino acid sequence as shown in any one of SEQ ID NO. 1 to 28; or, b) an amino acid sequence having at least 80% sequence identity with any one of SEQ ID NOs. 1 to 28 and having RNA cleavage activity. Preferably, the nuclease effector protein has 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more or 100% or more of sequence identity with any one of SEQ ID NOs. 1 to 28.

In a specific embodiment, provided is an engineered Cas13 nuclease effector protein. The Cas13 nuclease effector protein loses its catalytic activity through amino acid mutation. For example, the Cas13 nuclease protein undergoes mutations in a HEPN structure at its C-terminus and N-terminus (RxxxxH motif) to form the dCas13 protein.

In the context of this specification, the term HEPN refers to the two domains contained in Cas13a, which act on Cas13 equivalent to the HNH and RuvC domains on Cas9, to cleave target nucleic acids. The HEPN domain is required for Cas13a to cleave RNA targets. The RxxxxH motifs of the HEPN ribonuclease domain are located at the N-terminus and C-terminus of the Cas13 protein respectively. In addition, similar to Cas9, mutations of key residues in the Cas13a molecule can form Cas13a with missing nuclease activity (dCas13a), which can bind to RNA targets but cannot cleave. The RxxxxH motif is 6 consecutive amino acids, the first amino acid is arginine R, the last one is histidine H, and there can be any amino acid in the middle. The RxxxxH motif is the HEPN motif and has ribonuclease function.

In a specific embodiment, provided is an engineered Cas13 nuclease effector protein, further comprising a functional domain fused to the Cas13 nuclease protein. Wherein, the functional domain is selected from one or more of the following: translation initiation domain, translation repression domain, transactivation domain, epigenetic modification domain, nucleobase editing domain, reverse transcriptase domain, reporter domain and nuclease domain; wherein the nucleobase editing domain is adenosine deaminase, cytidine deaminase or their catalytic domain fusion.

In the third aspect the application relates to a polynucleotide.

In a specific embodiment, provided is a polynucleotide encoding the aforementioned Cas13 nuclease protein or the aforementioned engineered effector protein.

In the fourth aspect, the present application relates to a polynucleotide vector.

In a specific embodiment, provided is a polynucleotide vector, preferably the vector is a plasmid or lentivirus.

In the fifth aspect, the present application relates to a gene editing system.

In a specific embodiment, provided is an engineered CRISPR-Cas13 gene editing system, comprising: (a) the aforementioned engineered Cas13 nuclease effector protein or nucleic acid encoding the effector protein; and (b) crRNA, comprising a spacer sequence complementary to the target sequence. When using an engineered Cas13 nuclease effector protein as shown in SEQ ID NOs. 1 to 28, the crRNA also comprises a direct repeat (DR) sequence with at least 80% sequence identity with the sequences as shown in SEQ ID NOs. 29 to 56, respectively; wherein the engineered Cas13 nuclease effector protein and the crRNA are capable of forming a CRISPR complex that specifically binds to the target sequence comprising the target sequence and induce modification of the target nucleic acid. Preferably, the direct repeat (DR) sequence has at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99 %, or 100% sequence identity with the sequence as shown in SEQ ID NOs. 29 to 56.

In a sixth aspect, the present application relates to a kit.

In a specific embodiment, provided is a kit comprising the aforementioned engineered CRISPR-Cas13 gene editing system.

In the seventh aspect, the application relates to the use of engineered CRISPR-Cas13 nuclease effector proteins.

In a specific embodiment, provided is use of the aforementioned engineered CRISPR-Cas13 nuclease effector protein and the aforementioned engineered CRISPR-Cas13 gene editing system in preparing a medicament for treating diseases or disorders related to nucleic acid mutations in the cells of an individual.

In the eighth aspect, the application relates to a method of modifying a target nucleic acid contained in a cell.

In a specific embodiment, provided is a method for modifying a cell containing a target nucleic acid, comprising contacting the cell with the aforementioned engineered CRISPR-Cas13 nuclease protein effector protein and the aforementioned engineered CRISPR-Cas13 gene editing system, thereby achieving modification of the target nucleic acid in the cell.

In the ninth aspect, the present application relates to a use for treating a disease or disorder associated with nucleic acid mutations in an individual.

In a specific embodiment, provided is the aforementioned engineered CRISPR-Cas13 nuclease effector protein or the aforementioned engineered CRISPR-Cas13 gene editing system in preparing a medicament for treating diseases or disorders related to nucleic acid mutations in an individual.

### Example

### Example 1: Identification of novel Cas13 isoforms

### Searching for novel Cas13

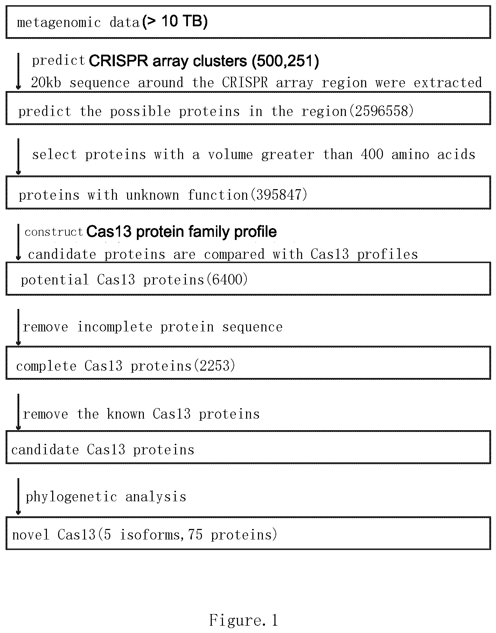

Nearly 10Tb (Terabyte) assembled metagenomic data, mainly from human and animal hosts, aquatic, natural and agricultural soils were downloaded from the JGI (Joint Genome Institute) database. approximately 500,251 CRISPR arrays were predicted by MinCED software, 20kb (kilobase) sequences upstream and downstream of the CRISPR array region were extracted as candidate sequences, and protein open reading frame (ORF) on the candidate sequences were performed by Meta-GeneMark 1, and 2,596,558 candidate proteins were obtained. Given the known size of Cas proteins, we selected proteins with a protein size greater than 400 amino acids as candidate proteins and obtained 395,847 proteins. The known Cas13a, Cas13b, Cas13c and Cas13d sequences from NCBI (National Center for Biotechnology Information) were downloaded, and a known Cas13 protein database was constructed by HH-suite3 software. 395847 candidate proteins were compared with the known Cas13 protein database through hhsearch of HH-suite3 software, and 6400 proteins with comparison results were obtained. After removing incomplete proteins and proteins containing less than 2 higher eukaryotes and prokaryotes nuceotide-binding (HEPN) domain proteins from candidate proteins, 2253 candidates were obtained. After removing the known Cas13 protein sequences, 1139 novel Cas13s were obtained (Figure 1).

### Identification of the novel Cas13 isoforms by Phylogenetic analysis

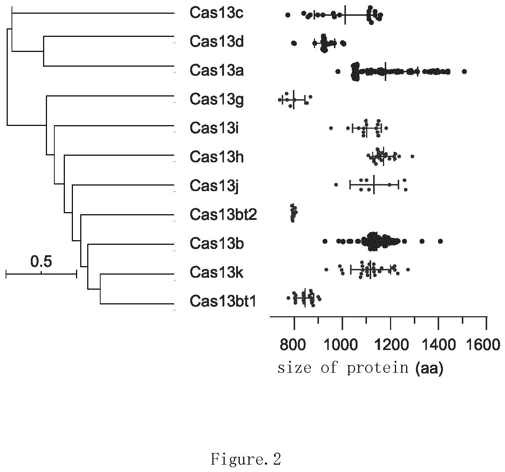

Phylogenetic analysis and classification of candidate proteins were performed. The 2253 candidate proteins were firstly clustered through preliminary clustering using the cluster--min-seq-id 0.5-c 0.7 parameter of the MMseqs software. Then redundant proteins were removed from each cluster through the cluster--min-seq-id 0.9-c 0.8 parameter of the mmseqs software. Each cluster was then subjected to multiple sequence alignment through MAFFT software. The alignment results were used to construct a hidden Markov model using hhmake of HH-suite3 software. Then the hidden Markov model files were compared with each other using hh-align of the HH-suite3 software. Alignment scores between proteins were used to calculate evolutionary distance, wherein sij represents the alignment score between i protein and j protein; sji represents the alignment score between j protein and i protein; Sij represents the average alignment score between i protein and j protein; dij represents the evolutionary distance between i protein and j protein. The calculation method of evolutionary distance was Sij=(sij+sji)/2, dij=(log(min(Sii,Sjj))-log(Sij))/2. Evolutionary distance results were performed cluster analysis using the unweighted group average method (UPGMA). Finally, many known Cas13a-d homologs and 7 different branches were identified. Wherein two of the branches were homologous to the previously identified Cas13X, Cas13Y, and Cas13bt, and we named them as Cas13bt1 and Cas13bt2. The remaining five branches are novel isoforms, which we named as Cas13g-k. Wherein the size of Cas13g protein was about 800 amino acids, and the size of Cas13h-k protein was about 1100 amino acids (Figures 1 and 2).

### Analysis of Cas13 isoform evolutionary tree

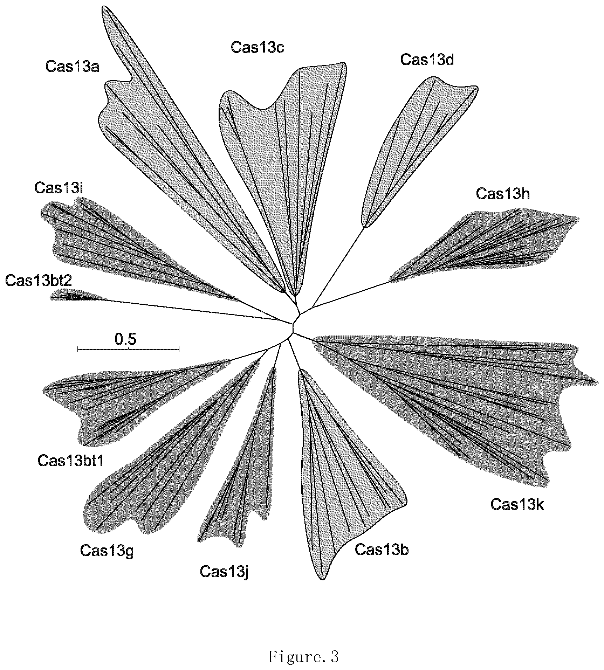

The novel Cas13 isoform protein and some known Cas13 proteins were subjected to multiple sequence alignment through MUSCLE software. The alignment results were used to construct an evolutionary tree through FastTree software, and finally the evolutionary tree was presented through the ITOL website (Figure 3).

### Example 2: Characterization of novel Cas13 isoforms

### CRISPR loci analysis of novel Cas13 isoforms

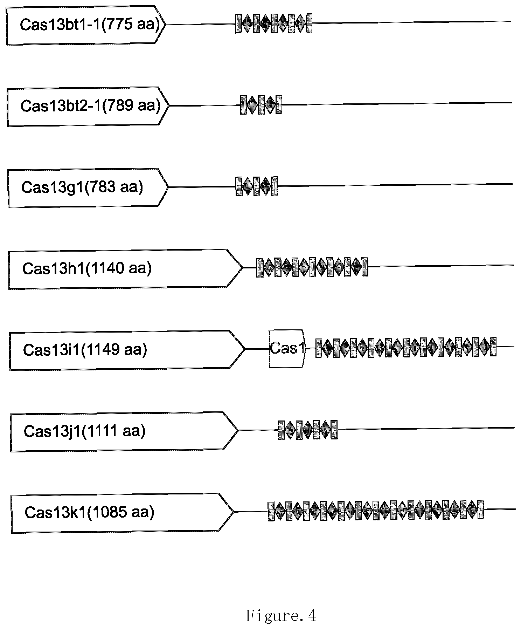

The 20kb sequence upstream and downstream of the CRISPR array region was extracted for ORF prediction, and the obtained protein sequences were compared using blast software. Cas13i had the CRISPR conserved proteins Cas1 and Cas2, while the CRISPR Loci corresponding to other Cas13 isoforms lacked these conserved proteins (Figure 4).

### Analysis of HEPN domain of Cas13 isoforms

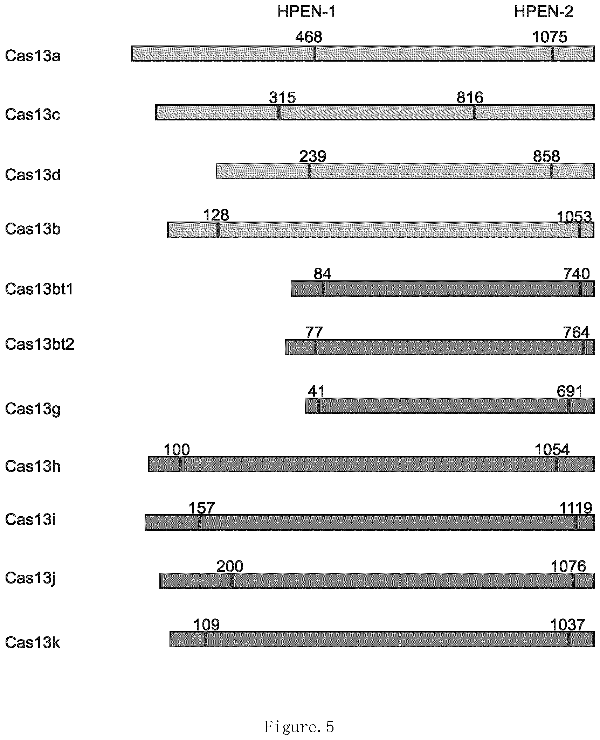

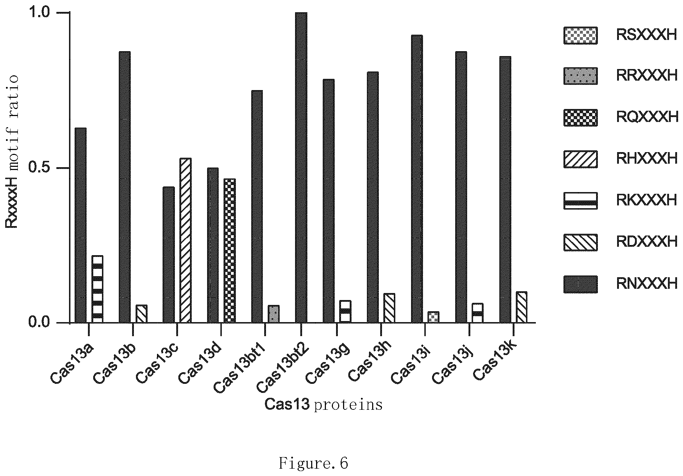

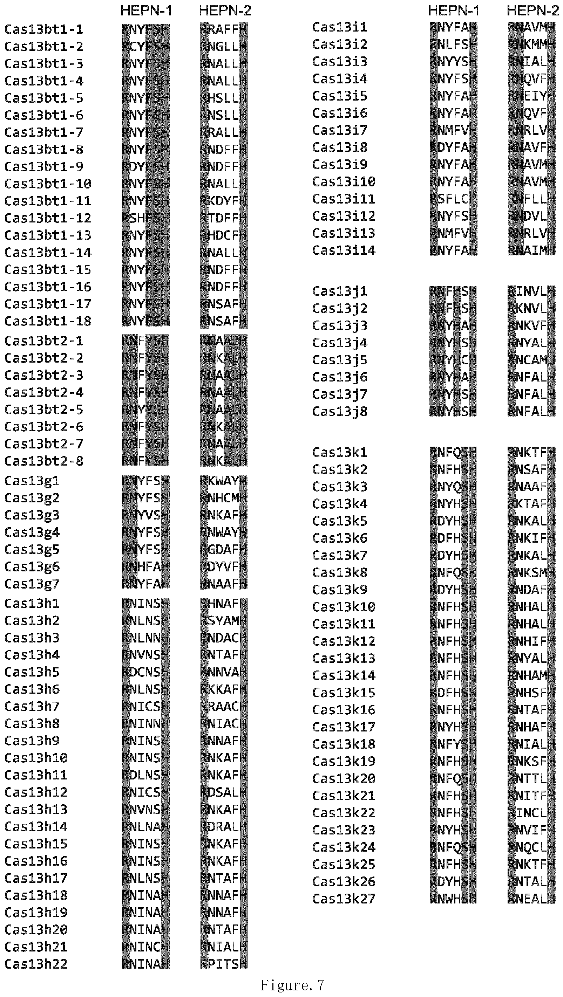

Multiple sequence alignment was performed on each isoform protein sequence using MUSCLE software, and the HEPN domain information of each protein was extracted from the alignment results. The distribution map of representative proteins corresponding to the HEPN domain in each Cas13 isoform was drawn (Figure 5). The RXXXXH motifs corresponding to each Cas13 isoform protein were sorted to show the proportion of the first two motifs (Figure 6). Finally, the conservation of the HEPN domain of the novel Cas13 isoform protein was analyzed (Figure 7).

### CRISPR array analysis of novel Cas13 isoforms

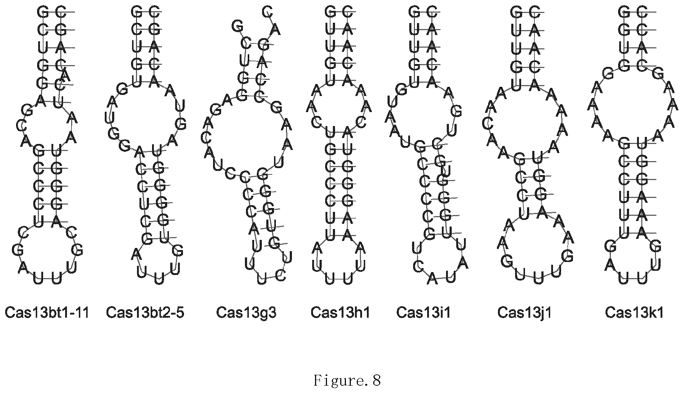

The direct repeat sequences (DR) were found by using MinCED software. The specific DR sequence information was shown in Table 1. The secondary structure of the DR sequence of the novel Cas13 isoform was predicted through the RNAfold website, showing that the predicted structure was also similar to the previous structures of Cas13a and Cas13b (Figure 8). The spacer corresponding to the novel Cas13 isoform was predicted through CRISPR target, and some of the sequences were compared to the IMG/VR database, indicating that the novel Cas13 isoform proteins may help microorganisms defend against foreign mobile genetic factors.

**Table 1**

| SEQ ID NO. | CAS enzyme | DR sequence |
|---|---|---|
| 29 | Cas13bt1-2 | GCTGAAGCAACCCTGGTTTTGCGGGGTGATTACAGC |
| 30 | Cas13bt1-5 | GCTGTAGAAGCCTCCGATTTGTGAGGTGATGACAGC |
| 31 | Cas13bt1-8 | GCTGGAGCAGCCCCCGATTTGTGGGGTAATCACAGC |
| 32 | Cas13bt1-9 | GTTGGAGCAGCCCCCGTTTTGTGGGGTAATCACAAC |
| 33 | Cas13bt1-10 | GCTGGAGAAGCCTCGGATTTGCGGGGTGATTACAGC |
| 34 | Cas13bt1-11 | GCTGGAGCAGCCCTCGATTTGCAGGGTAATCACAGC |
| 35 | Cas13bt1-14 | GCTGGAGAAGCCTCGGTTTTGCGAGGTGATTACAGC |
| 36 | Cas13bt1-15 | GCTGGAGCAGCCCTCGATTTGCAGGGTAATCACAGC |
| 37 | Cas13bt1-16 | GCTGGAGCAGCCCTCGATTTGCAGGGTAATCACAGC |
| 38 | Cas13bt2-5 | GCTGTGATAGGCCCCGATTTGTGGGGTAGTAACAGC |
| 39 | Cas13g1 | GTTTTCGTCACCCCCGTTTTGGAGGGTAAGTACAAC |
| 40 | Cas13g2 | GTCCGGCTTACCCCCCATTTAGAGGGTGTCCCCGGC |
| 41 | Cas13g3 | GCTGGAGACATCCCCATTTCTGTGGGTAAGCCAGAC |
| 42 | Cas13g4 | GTTTTCGTCACCCTCGTTTTGGAGGGTAAGCACAAC |
| 43 | Cas13g5 | GTCTGGCTTACCTGCCATTTTGCAGGTGTCTCCAGC |
| 44 | Cas13g6 | GCTGAAGACGCCCTGTCTATGACGGGCATGTCAGCT |
| 45 | Cas13g7 | GTCGAAGACGCTCCGTTTTTGAGGAGCAAGTACGAC |
| 46 | Cas13h1 | GTTGTAACTGCCCTTATTTTAAAGGGTACAAACAAC |
| 47 | Cas13i1 | GTTGTGAAAGGCCGCCGAAATGGCGGTTCAAGCAAC |
| 48 | Cas13j 1 | GTTGTAACAAGCCTAAGTTTGAAAGGTAAAAACAAC |
| 49 | Cas13k1 | GGTGGAAAAGCCTTTGATTTGAAAGGTAAAAGCACC |
| 50 | Cas13d_896aa | GAGATAGACCCTTGTTAACTCGTAAGGTTCTGTGAC |
| 51 | Cas13d_911aa | CTTATACAACACCCATTTTCACAGTGGGTCTATAAC |
| 52 | Cas13d_942aa | GATTGAAAGGATTGTAAATTTACAAGGTCTTAAAAC |
| 53 | Cas13d_957aa | GATTGAAAGCTATGCGAATTTGCACAGTCTTAAAAC |
| 54 | Cas13d_968aa | GAACTACAGCCTTAACGAATGTTAAGGTTCTGAAAC |
| 55 | Cas13d_982aa | GAACTACACCCGTGCAAAAATGCAGGGGTCTAAAAC |
| 56 | Cas13d_999aa | GTACAATAGCCCGTTGAAATAGATGGGTTCTAAGAC |

### Novel Cas13 distribution

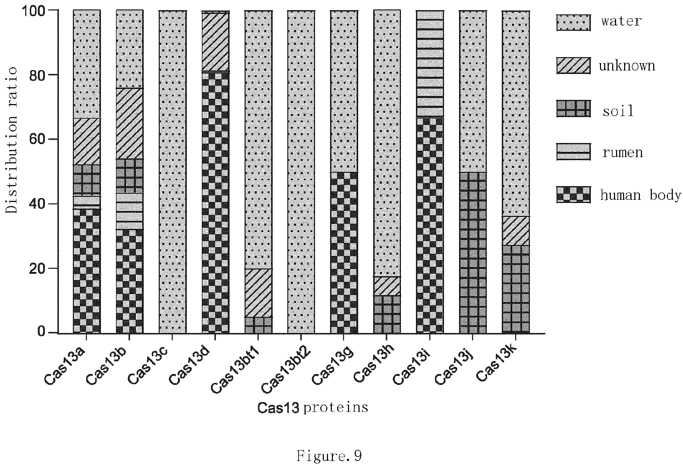

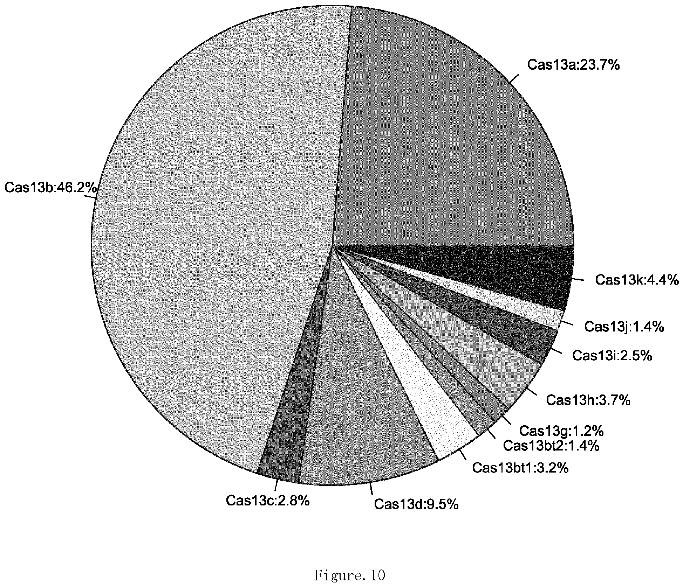

Statistics on the classification and metagenomic sources of all novel Cas13s (1139) found that 80% of Cas13d originated from human microorganisms, Cas13c came from water sources, more than 30% of Cas13a came from human microorganisms or water sources, and Cas13b was distributed more dispersedly. Wherein, Cas13d was mainly distributed in animal microorganisms, which was consistent with the Ruminococcus genus that was previously found to be mainly distributed in Gram-positive bacteria. Corresponding to the novel Cas isoform, Cas13i was distributed in humans or animals, and Cas13g was mainly distributed in methane-rich environments (Figure 9). At the same time, among all novel Cas13s (1139), Cas13a, Cas13b, and Cas13d accounted for 23.7%, 46.2%, and 9.5% of the total, respectively (Figure 10).

### Example 3: Identification of novel Cas13 isoform crRNA directions

### Plasmid construction

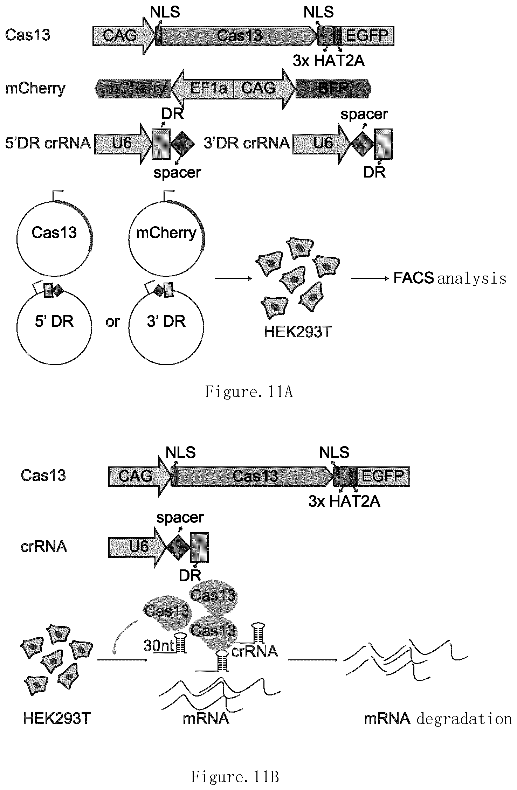

The coding sequence of Cas13 was codon optimized (human) and synthesized. The synthesized Cas13 effector protein sequence was inserted into the XmaI and NheI restriction enzyme sites of the pCAG-2A-eGFP vector to construct the pCAG-Cas13-2A-eGFP plasmid. The protein sequences used were shown in Table 2. The synthesized crRNA sequence (U6 promoter sequence + DR sequence + spacer sequence or U6 promoter sequence + spacer sequence + DR sequence) was inserted between the EcoRI and HindIII restriction enzyme sites of the pUC19-U6 vector to construct pUC19-U6-5'-DR crRNA or pUC19-U6-3'-DR crRNA plasmid. The corresponding crRNA targeted mCherry mRNA, and the mCherry spacer sequence information was shown in Table 3. The CAG promoter expressed eBFP protein and the EF1a promoter expressed mCherry protein to construct pCAG-eBFP-EF1a-mCherry plasmid (Figure 11A).

**Table 2**

| SEQ ID NO | Cas enzyme | Amino acid sequence |
|---|---|---|
| 1 | Cas13bt1-2 | MEKEQGLYSIDRYQGAGKWCFAIGANRAWDNYNERPKLFSESLL RYEKATRRDWFDEETRGLIKKSDVRQRLRKIRCYFSHYCHDNTCL GFDPDDDLRKIMEKAYERAIFEQRKHLSTETDIETPALFEPHGRITA AGVVFFCSFFVERRILNRLMGRIPGFKKTEGEYGATRQMFSKYCL RDSYSIRASDSNAVLFRDILGYLSRAPSQYYRHNKDQCDKDGHPE RKKDKFINLALRYLESFVPARLRNHTLSVGRKEVVRMETNAVAE GEGEYRPYPPKAKVKVVFTEDDPERPYYITHNTVILQTAKKEEDIH HCKVGVNELKYLVLLCLQGKAEKAVAGIEGYVRRIQGRFADHTN KVARDDDERLVRGLPEFVRVASGIETPDEVRELKSRLDHIRKKWQ TKKAESAEAQLHRKARDVLRHINWESQRPLGIEQYNRLLELLVNR DLESFAAEMKELKRRGLISEELLKSVEGIRNLNTLHVKVCNLVLTR LEHLVENDPEELKRHIGIVPREEKEGPSYEEKVRAFVQQPMMYRG FLRNSFFKGSGKSFAKLVEEELHKKGCPDVPLGTDYYLVRDLERD ERKNRFHNDNAALYETLALDRLCVLMARDCLVRLNRNLEKHAT RISWEATDAGDTICLELPRRDRDHESFRLSFGVRDYPKLYVMDDP VFLCGLMKHFFPDNQAIQYHELYSEGINKYTAMQAEGIAATLKLE EKTIKEKNMQIPATGYIRFCEIVSQSDFAPGEKRVLKNVRNGLLHY HLEFEPTEWAEFREIMKREGFDTAKKRKSTRKK |
| 2 | Cas13bt1-5 | MKIKNENTDKKTELYSIDKYKGRDKWCFAIVLNKAQTNLTENPD LFEQTITKYDRIRKEGWFDEETKKLIYIQENEHKIKGEIKTLAREVL KNLRNYFSHHFYKQDCLIFPKDNIVRIIMGRAYERSEYEIKKNLKE DISIELPALFEPEGKITTAGIVFFTSFFVERRFLHRLMGSVQGFTKTE GEYKITRDVFAKYCLRDSYSVKAQDNDAVMFRDIIGYLSRVPTES FQHIKNPKKQNESQLSERKTDKFISFALKYLKDYGFEDLKGHYTA FFARSEIKKEKEDIEIKDDKKHKPHRMKSKIEIHFDKTKEDRFYIER NNVILKIQRKGGRANILRMGIYELKYLVLLCLSGKAREAINRIDDY LNDLRNKIPHIENMNKEGIGEQIRSLPGFVRSQLGFVQIDDEKKKE |
| | | NRLDYVEKKWEKKRAESKELKLNRKGRDILRYINERCKKPLTIDR YNRILELLVEKNIEGFYHELEELRKTGRIEKNITQALVGEKNINALH IKICKLVQDELKSLEKEDLKKYIGLTPKEEKVVSFEEKLGRILDKPV IYKGFLRYQFFKNDKKSFARLVEEIIKEKTGGLDVPIETEYYSISTL GRFDKANKTLYETLAMDRLCMMMARRYFLSLNKILAKRAQNIE WKKESGKEFIVFKFNMPQDTGKSISIRFSPKDYTKLYVKNDSEFLA RLCQYFFPNEKAIDYHKLYSHGINKYTNFQKEGIEAILELEEKIIKK RKINSPENYLSFEEILNQSIYNDEEKNTLIQIRHSLLHYQILFSKNDL TKFYNVMKREGIEKIWSLVI |
| 3 | Cas13bt1-8 | MAVDYSLKQPFYQGVHKSCFTVPLNIAADNCKQKGYRNLLKEAQ RSKGGLSDQSIQEAADLIEKRLSAIRNYFSHTYHTDSVLTFQKEDP VKKFLETAWSYAVSETQKDIAESDYTGIVPPLFEDKEGQFQITAAG VIFLMSFFCHRSVLNRMFGSVKGLKRSDREQMGTGEKRDYQFTR KLLSFYSLRDSYAVKAEATRPFREILSYLSCVPHESLVWLSARGKL TEKEKKAFRHFLDPTVPKEALSEESAGDGSDSERPGVRKNNKFLL FAVQFIEAWSRKEKKGLEFARYRKSRVEAPGENQDGSEKRIVRFR SEIRDTQEDWPYYLRNNHALLRLHPGENKEPVDARIGEYELLYLV LAIFDGKGAKAIQKLANYIFEAKKQIQNARVYDRYQDLLPSFLTA GNKPVSAETIRNRLAYIRGELEKMLEAVQKEKKSGRWEMHKGKK IGHILRFLSNSIDDIRRRPNVKEYNRLRDLLQQLQWDEFDKALQSY VNEKLLDETVYRQLRGFHSLDELFERCCRLELKRLEDMEKAGGD RLNRYIGLEPKGKPKNYADLNTLQKKGERFLKGHQLSIPRYFLRN ALYKEYQATEERKPTSLYQIVRERLPRTNPILPDRYYLLEEDPKTY SGSDSKIIREMCFTYIEDLLCMRMARWHYEQLSEKLRKKLQWKE VQTGPAGYERFRLIYKISDELSIEFHPSDLTRLDVIEKDDMLTNISQ HFLTKKGTVRWTEFVSQGMKHYRDRQKQGIEALFKWEESLRIPE GLWKEEGYLGFEKVLEEAVKHGKIQDKDKEALKRIRNDFFHEHF CGTPADWEVFKRVLKRFLNQGKNEKKRFKK |
| 4 | Cas13bt1-9 | MPVNYSLDQDYYKGTHKSCFTVPLNIAWDNGSKKGCENLLKEA MRTRGGFTQEDIEKVHRSLAEKLNGIRDYFSHYYHEDKPLEFKKG DDDAVKDFLEKTFSYAAGETQKRVKESGYQGIIPPIFELCGDQVRI TAAGVIFLASFFVPRSTLERMFGAVQGFKRSDRGDLDTGQKRDYY FTRSLLSFYTLRDSYYLQADETRPFREILSYLSCVPFDSVQWLQAH GKLSKSEEKEFFGRPVEEQDEENPAQTEKQTAPAGRRMRKKNKFI LFAVRFIEAWARNEKLSVEFGRYRNIQNEEDRRKQSGKKVREVFF PSALNNLSAEEQDLEGLLYIRNNHALIRIHLKAKTPVTVRISEHEL MYLVLAILSGKGGNAVQKLSKYVWDVRMRSRGPLTNMPRNFPA FLRSPASEVSEQAVQNRLNYIRKTLKEIQANLQKEAQTGQWILDK GQKIRHILRFISDSMPDFRRRPSVKEYNELRELLQTLAFDDFYRKL ASFQTERKLDAAVWNNLAQCKSINELCERCCQLQQQRLDELEKQ GGDELKRYIGLLPKEKGKHYEEQNTPARKFERFIENQLSVPKYFLR CKLFVTGGSRRTNLLKLVQEHLKPKTSVFHEERLYLREEQPGDYP WSDRKIIQKMYYLYVQDLLCMQMAQWHYEHLTPQVKGKIDWEI NSESKESDGYNRFKVEYKGPQGCRIIFRVQDFGRLDFLNKAPMLD NICQWFLSGRKEITWPEFLRDGLQRYRQRQILVVRALFRFEENLKI PEEEWKGKSHLSFDEVLERFSGKNRLSEEEKESIRRVRNDFFHEEF EATPSQWRDFERRMSEYLNKEKREKPKKKKR |
| 5 | Cas13bt1-10 | MIENKPISQGSPQGKTVDDYKGEQKWCFAIVLNRACDNYEENHK LFSESLLEFEKTHRKDWLDEETRKLIHNVEEILPPDPQKKYEIKPKN LANIRLNEVRNYFSHFHHKDWCLYFKADDPIRIIMEKAYDKAKEK VIGHLKKEPEIKIPESLFESNGRITPAGIIFLASFFVERRFLSRLMGYV GGFKESEGEYSITRDIFSTYCLKDSYSIHTPDSKAMLFRDILGYLSL VPSEYYPTYLSQIPKRKPDEKLPKDEKYGERKPDKFILFALKYLEEI VSKGLADTYKVSVARMEIIREETKEAEKSDEQYKPRPNEGTVKIV FESKSKPDGEELPYYINHNTVILRIQKKGDKIHFCKMGVNELKYFI LLCLQGKTAEAVAAVDNYIHSLQSRFANPAETVRSDEAGIPEFILR QSGKVQDKDKEKAARIKYIRDKWEKKKAESAEMELHRKGRDILR YVNWNSKQPLGTNKYNLLLELLVKKDFDGFGKQLFNLKLKEHIS DEVFKRLTAFKTINTLHEKVCSFVLEELTFLEQNEPAKLEEYIGLV RKPAPENNPPPEYKDKVKAFVEQPMIYKGFLRENVFKENKKTFAK LVEETLGRLKYPDVPLGKDFYYVVDPKLSEKENRFQKDNKILYET LALDRLCAMMARICYENINENLRKSGQEIIWKKENDKEFLYLSINP AKLTTATLREPKTSRTAFGDNLKIPLPSGTQNTFTIRFDRKDYTKL YVMDDAEFLGGLVLHFFSKEKEPIDYHRLYSEGINYYTELQRQGI MAILKMEEKIVMNKKIPMTGNYIGFKTIMKESGYPPLEQNTLNKV RNALLHYHLKFEPTDYNKVVEIMKREGLESKNKIRKTDKK |
| 6 | Cas13bt1-11 | MGNISGEKIGIKMDNKKKGNNYSIENYKEDRFLFTAALNIAYDNC KQKGCLNILAECQHSKGGISDEQIKNVKDGIESRLRDIRNYFSHYY HNENCLMFEKDDPIKVFMEATFDKAVSNLSGSTKESDYKGIEPEQ |
| | | LRLFEEYDKKYRITMPGVVFLASFFCHRSNVNRMMGAIKGLKRA DRAEMDDGTKRDYNFTRRLLSYYSLRDSYAVKNEETRPFREILGY LSLVPHEAVDWLDSRGELSNEEKKEFLKEAKNQESKEDNDSTDE KTRRGLRKGNKFMRFAIMFTEDWSKKENLEVTFARYEKQEVHLE NKKQDGKKERNIKFPHEISASDDDWPYYIRNNHAIIRIKLKDKDAV SARISENELKYLVLLIFENKGKEAIQKLGDYIFDMSQKIRYDNYEP KDARRIPSFLKITRKEPTYEEVNNRLTHIRRELGKIIETIEKELKESK WLIYKGKKITIILKFLSSSIADIKKRFNVEQHDALRDMLQKLKFDEF YKRLSSYVGDGTLDKKTYESIQGIKDISQLCKKACELRLARLDELE KNGGSVLYRYIGLEAEEKNKEYEKLNTNQAKAERFLESQFSTGKD FLRESFYEQEREQKKSLIKIVKEQFANVVPMNEERWYLMNKNPK KFKDKDNKAIKALCNTYVQDILCMKIARWYYEGLSHAYKDKIEW DSTVETGGCGYTRFRLNYKTDCGVVIEFKPSDFTRLDIIEKPKMVE NICRSFITSNNDKKRTISWYDFNKEGVTKYRKQQVKAIERIFAFEK GLKIQDEKWQVQGYVPFIKRPEYENKGFKTFILEDAIQQSKIAEAD KETLNKVRKDYFHEQFFSSDEDRKVFEKCMPVVDDKKKFGKKNN RMYGKKG |
| 7 | Cas13bt1-14 | MIENKPISQGSPQGKTVDDYKGEQKWCFAIVLNRACDNYEENHK LFSESLLEFEKTHRKDWLDEETRKLIHNVEEILPPDPQKKYEIKPKN LANTRLNEVRNYFSHFHHKDWCLYFKADDPIRIIMEKAYDKAKE KVIGHLKKEPEIKIPESLFESNGRITPAGIIFLASFFVERRFLSRLMGY VGGFKESEGEYSITRDIFSTYCLKDSYSIHTPDSKAMLFRDILGYLS LVPSEYYPTYLSQIPKRKPDEKLPKDEKYGERKPDKFILFALKYLE EIVSKGLADTYKVSVARMEIIREETKEAEKSDEQYKPRPNEGTVKI VFESKFKPDGEELPYYINHNTVILRIQKKGDKIHFCKMGVNELKYF VLLCLQGKTAEAVAAVDNYIHSLQSRFANPAETVRSDEAGIPEFIL RQSGKVQDKDKEKAARIKYIRDKWEKKKAESAEMELHRKGRDIL RYVNWNSKQPLGTNQYNLLLELLVKKDFDGFGKKLFNLKLKEHI SDEVFKRLTDFKTIDTLHEKVCNLVLEELTFLEQNEPSKLEEYIGL VRKPAPENNPPPEYKDKVEAFVKQPMIYKGFLRENVFKENKKTFA KLVEETLGRLKYPDVPLGKDFYYVVDPKLSEKENRFQKDNKILYE TLALDRLCVMMARVCFQQINENLVQRAEQIDWKKENGKEFIYLSI NPAKLVIAQTQEPKTSRTAFGDNLKIPLPSGTQNTFTIRFDRKDYT KLYVMDDAEFLNGLMQYFFPKEKTIDYHKLYSEGINHYTELQRQ GITAILVLEKKIIDREKLPTDVKYIDFRTIMENSGYKREEQIALGQV RNALLHYHLGVEPKENNKGYKGFKPDDFKTFVAVMAREGIRKRE KWNLKI |
| 8 | Cas13bt1-15 | MGIDYSLTSDCYRGINKSCFAVALNIAYDNCDHKGCRTLLSEVLR SKGGISDEQIKSQVVDGIQKRLKDIRNYFSHYYHAEDCLRFGDQD AVKVFLEEIYKNAESKTVGATKESDYKGVVPPLFELHNGTYMITA AGVIFLASFFCHRSNVYRMLGAVKGFKHTGKEQLSDGQKRDYGF TRRLLAYYALRDSYSVGAEDKTRCFREILSYLSRVPQLAVDWLNE QQLLTPEEKEAFLNQPAEDEGGDISDSSSSDKNKKSKEKRRSLRRD EKFILFAIQFIEGWAAEQGLDVTFARYQKTVEKAENKNQDGKQAR AVQLKYRNQGLNPDFNNEWMYYIQNEHAIIQIKLNNKKAVAARIS ENELKYLVLLIFEEKGNDAVQKLNCYIYSMSQKIEGEWKHRPEDE RWMPSFTKRADRTVTPEAVQSRLSYIRKQLQETIEKIGQEEPRNNK WLIYKGKKISMILKFISDSIRDIQRRPNVKQYHILRDALQRLDFDGF YKELQNYVNDGRIAVSLYDQIKGVNDISGLCKKVCELTLERLAGL EAKNGSELRRYIGLEAQEKHPKYGEWNTLQEKAKRFLESQFSIGK NFLRKMFYGDCCQKRCFDEEKGYNTQAKERKSLYSIVKEKLKDI KPIHDDRWYLIDRNPKNYDNKHSRIIRQMCNTYIQDVLCMKMAM WHYEKLISATEFRNKLEWNCIGQGNMGYERYSLWYKTGCGVVIQ FTPADFLRLDIIEKPAMIENICQCFVLGNKKLNSGAEKKITWDKFN KDGIAKYRKRQAEAVRAIFAFEEGLKIQEDKWSHERYFPFCNILDE AVKQGKIKDTGKDKEALNRGRNDFFHEEFKSTEDQQAIFQKYFPI VERKDDTKKRRDKKQK |
| 9 | Cas13bt1-16 | MAVNYSLREKWYRGVNKCCFTVALNIAVDNCKSKGCETLLKEA EHSKGGITDEQIQQSYTEVEKRLNDIRNYFSHFYHGDECLIFKKDD IVKRFMESVFATAVSNVVGGTKESDYKGVVPPLFEQSNEDYMITA AGVIFLASFFCHRSNVYRMLGAVKGFKHTGKEELSDGQKRDHGF THRLLAHYSLRDSYSVKIEETKSFRDLLGYLSRVPQQAVDWLNER NELSEDEKKEFLNQKSSEEESPEQPEPENAEWRTEKTSRRSLRKTE KFILFAAKFIEDRAEKEKQDVTFARYQKTVTKEENKNQDGKQAR VVRLKYEEDKKDDEKPREHFNLEWMYYIRNEHAIIQIKPKDKEAV AARISENELKYLVLLIFEGKGGDAFNKLSDYIFRMTQKIKSGQINP NEARLPSFLKNPVKNITDKMVRNRLDYIRGQIKDVLEKINMEEPQ NNKWLIYKGKKISLVLKFISDGISDIKKRPNVKEYDTLRDTLQKLD FNRFYERLKSYVSDGRLAAALYDKIKGIDDISELCKKVCELMFAR |
| | | LAELEKKGGFELYRYIGMEVQEKDEKYDEWNSPQKKAERFLESQ FSIGENFLRESFYSEYCQKQECIDKEISLNTSVKNRKSLVYIVKEKL KDIMPLHNDRWYLIDRNPKDFERKDSKVIKGLCNTYVQDVLCMK MARWYYGQLNPALKNNIKWDETGQGHGYDRYKLSYRTNFGITIE FKLADFTRLDIIEKSDMIENICRSFIKPNRTISWYDFKQDGVEEYRK RQYKAVRAVFAFEESLIIPGRDWLSQGFVPFIKNEEYVKKGFSLFV LDEAVRQLKIKGSDKDAMRQVRNDFFHEQFQAKDEQWKVFEGY LSCFMIDRPKGEKNKKRYNGNKK |
| 10 | Cas13bt2-5 | MNMDTIELKKEEAAFYFNQAGFNLRAIESNVFDAGKRKTLLENPQ VLAKLENFIFNFRDVTKNEKGEIDVLISKLTDLRNYYSHYVHTDN VKVLSKGEGPILARYYQFATEATASTNVKLEIMDKGNKLTDAGV LFFLCMFLKKSQANKLISSISGFKRTDAEGQPRRNLFTYFSVREGY KVVPEMQKHFLLFDLVNHLSNQDEYIEKSQQTYDIGEGLFFHRMA SKFLNTSGILRGMKFYAYQSKRLEEKRGKIEPEGDSFVWIEPFQGN SYFEVDGHKGVIGEDELKDLCYALLVAGKNANEAEGKITQFLTK YKKADNSQEIEKDEMLCIDNFPANYFDGPGVGSIKDRVLNRLEREI KSHKDNKADSKAYDKMKEVMDFINNRLPAAEKMKQKDYRRYL KMVRLWNREKGNIEREFKAKEWSKYFPSNFWRANNLEDVYKLA RQENARILGNLKAVVEGMSEQEFEKYRQINEAKDLAGLRQLAGSF GVKWEEKDWEEYARQIKERITDRQKLTIMKQRITAELKKRHAIEN LNLRITIDSSKSRAAVLNRIALPKGFVKTHILQTPSDKIMKKTREAE CKILLSGKYGDLSKRFFDEKNLDKLTQINGLYEKNKIIAFMVVYL MEQLKLGLKGKTKLAELKETRIRYKISDKVTEDIQLSQYPSLVYVI GRKYIDNVDRYKFAGDVRGKPILSKIDLIEKERLEFIRQVLGFEKEL FDNTVIDKKNFTDTETHIKFRQIIDELVRKGWDATKLNKLKEARN AALHGEIPEGTSFQEANVLINELKNKK |
| 11 | Cas13g1 | MDICGRDVFLKRYGGACKNQEERKKQQKFFNEIKNFLKTLRNYFS HYLHTKEKVESLLKEEKCREFVSFLRRKALEVWNKRFENSALFSE MQAFSQNGWEEMKRELKESGPFLTDVLLFVSVFVPRGEMMRLLD AFRLSPDREKRECRREILSALCLSESYDFWSVDKKAGIALTILDHF ARIMGKRTDKAGKLIMSAPRKPENRKETPEICRRFRPSFFIRMLVG FIEEENVLKGFEFARSYNGRPVYDESKKELYLCRNNTRVRFPDNE GNLLESELGAEALKRIVLLYLKNLKGNDLAPLVRGKIKAIQKTLPQ RKNGTNAALLQRVKSRRDELSIRFFRKGLSDHQKAVAVLDFMNLI FPADKKFGKQAYQEALRALTGRYDEEAFLAVLPDIKCSETPFPKR KKPQAWARGAGSLETLHKRAEALFREKAGSLCEETAEEIGRLFHV RALKEKTGEPFERFKKLLTPPPRAFTGNGFGLTKEELLKDIPLEAEL YAPGGGPMASAVKKIYDNDRLLLKLARFILEQIREKDKVQVQITE KPDYDYEALVTVGEKFQVIIDSAQARRSHVSLNTERLKNVIQNYW KREETPVPFVVPDMRKREKNTPPRCWTEAEQDMERERRTMVEAL LKWEKYLANEFAKENKTATGNIDAIIKLMKPEKGEYVDFPLLLAR MNIPEASEYNDFRKWAYHQAPKKAFSDAPGELKERFLKRQAEKD RKRREQKKNSIKKVQKK |
| 12 | Cas13g2 | MNKRAENKINFFKDNFTLGQYAAFALSFNQAGRQLEKLSTTYHF QRIYGRRRDKVPSFKESLKNLRNYFSHCASQWPDEERFKKSVLRN ELDFLVLEAIAILEQRNTKLHDNDETAKDIKKELDKIRSSPSDFFPL DNSGKNIQPTLALVLSLFLTKNQMAFLLGKIFRGNGVTRESPQYL AQAKILETLSQNDRTLVDTDSQSERFTSHDKEFGLAIAGRLEAVGL YEEQQKIEDFPEDVWFIKQLVLYLEYMNVLPSVQFCRMTTIEKDD QLLQEKDFDIAHREKPLRIRCNTVEAQVKLSNGAPYTTNFGVQSL KYLVLAHLKKTFEDKEIDQLVVRQIESNPSRKCRKAKESGVTGDR LTKRIEYLIGKHTPSGENPVRLYEQIRFICRFVNEAWFKRYNRHMN SEEFKDIQERVRHYRRDDFQKLLKESDLLDIAGLNLGAGNDKKLG SCFRHERIQNIFCEMESGYLEWLENRKQQIDKLSQEEREALAARIN LRHREGRSSQEAFRPVSISADVLRREIESRDKAKNKRRFFDHIRDF GGSARFFARFDLDDRGWGSARDRERWARTGLLSQIVLKSLSGANI KNMLDQKPSQWECEERVDGTKITFKLTQGWRYYAATTKTQLRK LIGAYRPDLSVLPLLDDGGIEGGSVQSLKRTLERERYRLMQAILV WEREIIEKHDMKPDGGYIKFESILEKGATAEQAPDLKDIRNHCMH GDIWKAPFSQAPEPLRRVYSGLEEKSREKRRSQKKSGIKRSQKNQ K |
| 13 | Cas13g3 | MRGGNKSANEGKKFDFNLKQQAIFALGSNWAQKQFELTKKTQHS HRLREKLSIFQEGDIGRLEEKIENLRNYVSHGAHSGLAPLDSDEIA AFEAIVRKAVTSYLAIPREAQNIKEEKQTKIDKIRARMKKDKPLLS FSTGPLHQQNHPELMFLLAHFLTRKQLSYLIHRVYWPKEREDDKE PIQELLLFIAQPDTIIMRSKADEDARDTWISAEEEQGFAIWNYLQK RHADEVYTAPDDHYVMRQLVAFIESHQILKGAIFMRVEVRPDEK KEGAYKRVGVYEKQGSDNSLPLNIAYNTIRVSFVEDKVEGTFSLK TLIYITALFIGRVTPDKLTDFLITELKKNRDYSHRPSPAAKEGGDIK |
| | | TRVEKRLCYLLRRLNKPPQNLQEQIRFICQRINFAYQQKYGQYLD QNDYKTLENLVRYYRKPDLLSWLEGNAIGQQSGIHMGQEDSKTL NQLIKASSIEQLYLDMKSHYRYGLKYVAKNYQNWPEDKVADLA NIIGVRQQKTVSGNLPNAPVGVKLSWILTEFQDEIEEFKGIIHMLSA QYAPFKFEKPDKKFKKPGDAPRSEKNKRGWAHAKPFVVKQLLLN MAWYNVKEISDNDARLAGGAFVPISDISFKRSFSGCSLRMSFGKS WRQHARKSREYLQGLIDSYGEGRREFVLSKAEEENGISKSIERMEE EARQERLLFIQAILDWEKGWLKKNKSEAERLKTAKGYVEFREIAE KESLPDTIKELRNKAFHDGFLRDTKFSDCVEPIKSIYEDLKQKHI |
| 14 | Cas13g4 | MSEDLYRSDNLFCLSLEQQAALSIALNVALRRCREQNDSDMDICG RDVFLKRYGGACKNQEECKKQLDFINEIKGFLKTLRNYFSHYLHT KKKVESLLEKEKFRDFVSFLRLKALEIWNKRFGKSSLHSQMQAFS QNGWEEMERELKERGPFLTDVLLFVSVFVPRGEMMRLLDAFRLS PDRKKRELRREILSALCLPESYDFWSVSKKAGIALSILDHFARIMG KRTDKAGKLIMSAPRKPENRKETPEICRRFRPSFFIRMLVGFIEEEN VLEGFEFARSYNGRPVYDESKKELYICRNNTRVRFPDNEGKLSESE LGAEALKQIVLLYLKRKQGAFKGNDLAPLVRGKIKAIQKTLPQRK NGTNAALLQRVKSRRDELSIRFFRKGLSDHQKAVAVLDFMNLIFP ADKKFGKQAYQEALRALTGRYDEEAFLAVLPDIKCSETPFPKRKK PQAWARGAGSLETLHKRAEALFREKAGSLCEETAEEIGRLFHVRA LKEKTGEPFERFKKLLTPPPRAFTGNGFGLTKEELLKDIPLEAELY APGGGPMASAVKKIYDNDRLLLKLARFILEQIREKDKVQVQITEK PDYDYEALVTVGEKFQVIIDSAQARRSHVSLNTERLKNVIQNYWK REETPVPFVVPDMRKREKNDPPRCWTEAEQDMERERRTMVETLL EWEKYLANEFAKENKTATGNIDAIIELMKPKKEGYVDFLSLLERM KIPEASEYNNFRNWAYHQAPGEAFSDAPGELKVRFLKRQAKKDR KRREQKKNSIKKVQKK |
| 15 | Cas13g5 | MIDSFKDGFTLKQFVAFTLAANWAFDQHNNDQHNNLINTQHFYN LVKKNMKFKGDEDIFRGEEEAESDAPPKSIIKSIRNYFSHFIHSPLRS LTTVEARDLNDMARRLLVELNERNIKANDKDDIQQHIETYLDEST SLFTFEPKPILDHAQPEMAFMLALFLSKGQMAFLCGKIFFGTDARS KQKDGTYKDTPKTCLQKKLLNMMAQPDNIIRRLQSDDTLDPWLE SKHEQGFAIWQLLASSQKETAGGNEPDYLKNGNYMIRQLVRFIEL HNVLPSYEFARIETVETLSDDVKKLEQKIQFSSNPDLPLATRHNTIQ SVNQQGIKGTFGVRTLIYIVVVFLEQKRAGELANFVTTWLKANAH YPKTNLKGKDRPLSDQIAKRLDYLLKETNVKSKTNAKKSLHMQIR FICQRINHVWSLKYERHLSVHEYGELEKMVRYYRKAELRGWLAD HGLLDIQNIQLGRGGIKTLKKAIKAESIQQLYVDMLSDYHCWLQE QKDKLPKLNDAAQQSMAKAINVRNNRQSNNKPDFPIGLPEKVLR QKFFVVDGKSHARSLTTILKSIPTPVTFNEPQGTKSKALKKSMQIR NYQQLLLAMAWHAVKDLISDERIKQEKNSETLPDLAEIPISINCEN VRISMKFKKSWRNMATLNKGYIGKLMKQYCAGEKTIPMFRADET KAGKSVETTMQTFHSERYRFMQALMQWEKEFYNTNPALAESSDE IYLKFECLAEKANLADGIVSMRGDAFHDGVPNARFADCPEPTIKTI YDQICEREKDKQKTQRQQGVKRNLAAKKNADKS |
| 16 | Cas13g6 | MNHDVAHSHIKDDVTLRQAAAFTLASNWAHQQHKGVLASVHGE SLYRYALPVDEETIAAYRNHFAHLHRPHMPRLWQEPDVKDRLHT LVKEAARMMTMREGKIWDDDEKQHNAIHTILLRIENDPAYGFDL DRPQQAWLSITLILAPFLQKGQMAFLWDKMVEKRGDADEALDK ARFHLLKFLSIADSALAITPQQEEERLFSAQTEHGLALLVRLKESLD KITQDESSQATHAFPQGGYVMRQLILFLERTNALPSVMFARTRTH LEGERDGKPLLRQERIFVRERDAQTHDGMPLPPLKIQANTIRVQIQ EPKTDKQWQGMLGIHTLSVLVCAFLLGKDVDRLVLSWYREHGD KNDWRQGKEAKSPTPERIGKRIDWHRERMEGATTLYQKIRMLAD LMHESYREKHGVAMSTSDYRDLLYHMRHYRLHTLKAMARQDY GIETMNVPMTGKPWSFLLKKQDLDAQYDDIVAYRCRWLSDVKE RCHRMDDKERMTLARALGVRDVSPKDVNARGKNRVALLPAGMP VDAVRDAFPEFRAGHPDDKHAKKRHFIDVMRDNKTLKQGGGIPT QPFGFTWDTDKANKDKADKDKTCAGGWRAMTKRRSQWAHASL LHMMARHLLQGDYAVETPHDKKGKATSHESNKKKQVALGEMPF ASVKPSALGMTLEVEQGKKVLCSLTQGWRYYARLDKKTIKNLVT AYTKAGKGRTWTLPLLRASAGDKEESVEQAVTEMRRQRFIAAQA ILQWEYDVLKGKDIDKDKRLDFPAILTYAGLHDDSIRKKLTHYRD YVFHDNVLEHPFRDAPDILRECYKKIEAEEKKRRQQKRRQHIHQR PVQHSGKGKGSKKHKKTR |
| 17 | Cas13g7 | MQDRQVKREPFKDGFYLKEYAAFAIAAHNAHLACIKCATSILETE FFAKRYFPLLKAAIAHHPDFTPNAGQASWPQREELASFTKSDRAQ KYNQAFSKCLTEYAGGIRNYFAHYLHTLKPLQLPAEDRVFCFFLE ELQKKAVAVWKARHAAWRAKQSPHGEQYDSILECSQRPDFWDP |
| | | LTVQRDDCTILSCEAILIFLSILLPRSRMYMLLDKVLVRGESGTETE KKRISKRELLTALSPRDGHSIRIDPERKSLTAAFTVFNHIGTRYGRL TDTDGNLLSPRPNNIDDIVASHHTVFFIKMLVAFIEGEKCLPSFEFA RSYHGKPVFDQPDRELYIRHNNVAVRLRDEPRQQAVFSIHLLKLII LNLLSDKGNQTPDVDDWLKKQLRNFRSRAGQGDHSATPNSNLER MTPSFMKGEPGRPPKQACSLRKIVENRLQLLQERWFGENAETLLP HQMAYAILRAVNLMVPPENVLNSLQFREALLALTAIPYSADAVK GALPDIPRRKLNVGGQTLRGRLSGSQNINAAYGKIRSGFSEYCNE MMDLFDDQETTALRTFAARIGVQYIRKENSLEHERHKKDREQYQ KHYLEKLVSIPPVLFVRQFMDDDAKNVCFGTLLQRSRYIREVREA GLWDVKTLLADGQKFRDKHKEVINRDQLLLALVQYVQERNLKA QIKTGKKKKQGNITSLFDLSEWKSSFAFRVEGGKSVVVDTAKAW KYYLRTDPAWLQKVFKTYCSEIQTDTLPFVGPPDLPRRKENTPPRS WREAEGDMEQERYILMRSLLIWEKNTGSSRAKGEEYKPFKDLVT EAALPNGDEVTSIRNAAFHGVPDKPFRDAPEPLQQIYQQERERLQ KKRGEKKRKAQKN |
| 18 | Cas13h1 | MENTSLRTFKRFFDFKGSVAPIAEKANRNYALKKRNNVNLQQRL HYFAVGHAFKNIDVENIFRAELDEETKGKKPTKFLALQLSNFTFIK ELGCLLSNIRNINSHFIHDFELIKLDKIDDKIIEFLKQSFYLAVIQTCI KEKETTYIDFISTKDYEKQIVNFLLEKFYPFNDKRKKLTEEEEKRV KEYKSFRNDFKNKSIDEAIDSILFVKVNETVEWNLFETHNVFNITS GKYLSFEACLFLLTMFLYKGEANQLISKIKGFKRNDDDKYRSKRN LFSFFSKKFSSQDIDSEENHLVKFRDLIQYLNHYPTPWNKDLELES ANPAMTNKLKEKIIEMEIYRSFPDFANDERFFVFAKYQIFGKKYLG ENIEKEYMDNSFTAAEKIAYEYEIKISPEIKDANNKLKELKAKQGP YGKQKERNEKIIRELESMIKDGKNDPNPITEKLKTRIVKNLLYVSY GRNQDRFMDFATRFLAEEKYFGEDAEFKMYKFFSSEEQENNISTL KKELSKKQYDNLKFHQGKLVHFCTFDEHLKNYESWDDPFVIENN AVQVKVYLSNGINKIVSIQQNLMIYFLEDALYCNENVKELGKTLL TDYYLMHKEEFEHTKLFLQQNPTISREDKTVFKKILPKRLLHHYSP AMQNNLPEFSTFQLLFEKAKRLEERYNKLKNKAEDEGNLDDFLK RNKGRQFKLQFIRKACHLMYFKESYLRQVQEAGHHKRFHISKDEF NDFSKWMYAFDETPKYKDYLRELFSQKGFFDNPAYKKLFEDSVS LDTMYLQTKKNYEEWLKTFVPGQRATDKYSLNNYVKFFDNDLFF INISHFIHFLESRNKLQREENGNIIYHSLSNSEYLINEYYFKDKLEKS EYKTCGKLYNKLKTIKLEDSLLYEMSMHYLQIDKSIVKNARTSIIN LLVQDVKFSIEDANKNHLYDLFIPFNKIDSFVELIKHKEEQEQDKR FGGNSFLSKLSSYIEKVRNNKDIKPIFDRFIKKNSLNFDDLNKINNH IITNSAKFTKVELSLEEYFIFKDKILIKKENRINIDEIKNLSKYFNKV DRHNAFHFNVPNECYDIFLNKIESTYVKDEVRPVAPKQYPDLTKQ QRSVCSTFLDAIHNDFFNRSDREKMREEAENKYFQKIILNRMN |
| 19 | Cas13i1 | MSIKITVGSSNDGFTRHETKWHDSDWDENDDSKVPRCIDIGGSRIG ALTEHLEIVVPKDMGTKKKKDEVKDFTLYLNYAVSNLREITGIEG DEESKIKERLENLSDEKKQRLADFLWAFRFDEPQRDFAARGNDQK QFDHDYKRLAPLVATKIFELRNYFAHLDRRGNEALVCDRELYVLL EGILRPLAEKECMGPGCQTSKLYKMHLLNLRGPRKPPQPMESRKY DLTRRGVMFLVCMALYKDDAEEFLSCFADMRVPNRRRDSLDGLS ETDIGKLGRKKPSKRAFLKAFTFFSYRRGRVSLDGEDPDFLNFANII GYLNKVPGASFDYLALEDERKLLADLASRSTESEENKLFKYDLSK QVRAKDRFVSLAAAWCERFDVLPCIHFKRLDITPSLGRHRYLFGK ENDNTVHLDRHYIIENGAIRFEWRPTTHYGDIKIGYLRSCIGEAEFR RLLFCALRKPAETNAMLDAYFTAYHKILELMLNAPALDSFSIFSND ELVEAVQTVTGLTADELADIPEERLRQFFPKNLWRFFFAWEASQD DDDLRAAVAAKIDGRIKWCSDFLVRVENFRKWKAENFWKPNDQ RGPQGACPKSKLVNPPRNCRTSDASCVARVIAYLNYHLAPERKFR QLSLGKQHSHPGRNDNDTHNYEYQLVQSAIGKYALDQTSAVGGT KNGRVIKGTVAHLRPELELHLAQLRDKVRALARENLQPMEDSLR AAKRKNLLSGFACLDADKALKSARNSRKLIDLAEAAAELLKEELL KEAESLLSVGGDALKVLCRKFGVRTGLPLDKDALVKTILGIDMDK WTHAFDYGHGHPYVDRKLTEGDHVAAQIPLPNGMAERVMRSQS RVFDGLIGESGIDWAKAFASLDEKNISLLGFYDVSPLVGYIKTHSD HTEEEPDSSAPGINTWADADSRTIKPDFSRGGINKAIRAIKTAHNQ DRLLLKFAIQHWEEFKKTRAYDERKTAYEKALSVRDYFNGTFTY ALKGGVGLRLSRNDVFSPTFTHVVENAAAIVDLLKHENPDVKEVS FYDVAQLFARKQRESRKLRMELLPLIEQFGAKSPIPQEEYTRINAE GGKNDERKKKIRAMEYSYYSKAMPTLTEAEYNLVADVRNAVMH STLLVTDDDAYRAAKAVLGRLVSAR |
| 20 | Cas13j 1 | MDTPSKESSGKIIEKLDELKKATQESIEQRKKFDYRIGKGLADKLG NEKDSYFEKYLRKTVGENIPPKILVKPKGGWKTETKGKGKDWGD |
| | | KITFAAMPQHHYNTLLAMAFTKAHKVYSDIRGKVEIDAAQYETK AKLIEVEYGKDETRGLSGLEYLMMSKHLFSGKNSNIKLAVKKGET EILKEYALNEKLIYTVLDELRNFHSHIFHEPGPVSFKNLYGDEYKP EKKLTEEEWAIARDWFVNRFNDAKEHKLKTLAKVLEREGTTEEK EDAEKVIKTISGYSFEYNNCISREALLFIACMFLRKSDAAYFTKKW TGMKKAEGVFKSTQSFFTDNALKESKSILTLNADLYKYRQILGVL STMPAMKTDSLKPFYDFIKINNDSYSEKAEKARSKEEKEKIQAFIIP QRKSSNYTYWFMKYLNDNKLLDGFRIAYYKTPEDRFMYLIHNGL ISQDDLENIEDFKTPDEKLKYLREKGFNLKLKMKQAVGDEKKSLT EIYKETQRNFVFKVPTIENDNFCVKKLNVFFQTDIEFNGQKISVQLS VSPDFLMKWVFVLLITGEDSIKNAITEKKEKIKDILKKYAEEYYNR CITSNFNEPLMGLEASKVFPSSLTSTVEIDEKIDKDKILMRISEKYN ELTKFDEENKSRKAPWRFASKRKIDIILDYVHLVYSDRAFDEKKS VDAMRHEALNDMEYMDTFEYLRYYGRYRETEEFKKIFFEDKKLY FSPILKAMKQLDSLEGVFNFAITGFLNYLKGIQSKVTDENTNKYGK VFKVTGKSLTSKIGHHSEMFSVNHCVPQELIKLNDIKGYMKWKHE TKDKLWISDFAFIRNVLESRGGFSNTDYLMKEVMPLITFEKNEKGS IKGNTQMFVALSRNKTNELMLWEIGKYYWKEATGSEFSRLFKGL EKNTGNKITKAYRFTNPYYTIYQEDLDIKIQRKDKKGKVIVNSPVY TIKIKPKKFDDEYQYYEQEHIVDYIENYEPKKGIDGHWHFEELNK KIKDELARYLDDIYLLMTVEKHIVQKDFDKYASLLKEKDSKLPPY YIGFKRNDIALNKVIFDALKHKGSFSADDLNIYRINVLHQILQPKRE KYIIIRKSLIEYCAENKLLKTM |
| 21 | Cas13k1 | MKKELNREDVFTGGKTPELTIYYNIAYFRMAGLINALCGNKLERD KDALEQFMKAFINGKQKLTDMQFVKLCDYLWKGYKYDKNRSEY SLTENDKTMVLKMVAKLQDIRNFQSHIWHDNKVLVFDADLVQFI KNKHLEAIDAQRQRFSKETEVYEKENRELKKKKNKELLFDIHDGL GYITGEGRNFFLSFFLTRGEMTRFLKQRKGCKRDDTPEYKIKHLV YRHFTHRDGATRTHYGYEDNMLDQLPDKQEFLTTRHTYRLINYL NDIPEEVTNPELFPLFNTISNGILIKESVGTYIAFINKMPLLSDFEFSE VWGKDGKPYTNLVEFYHKTQPGFKFRTDLNSFHKIILNLIRDENY TGFFTTQLNLFMAHREELILVISKPLITPNDLLLIEEHYRYKLKAND FVREKLGEWKEKIEKGKWEKANEQKDKLINLLGGVIEITFYDFYW QKDEKPRNENRFMEFAIRFMADFNLLPDCEWEVEPLQIENDLIAN RTATPDLKKSGSQFMNQLTDNYRLKFNDGQIAFRYQNQVFIMGH KAVKNLLIACFDGKEKMLNGFMPALLADLKKITNEHILKNHQAL NTLSLLNNDSIPSYISRQWGEAEPITDMKKKAIARMDYITQQFEALI ENHHYLNRADKNRQIMRCYKFFEWQYPQNSQFKFLRRNEYHRM SIYHYCLDKEQHKYDKKGHNYLYNRLIKESHNESGNIEQHLPYQI RTMLNDAKDFNDYFLRILNATHKILTDWKNQLKQGREPNNYYLS RLGFTGGLTQKVVHTRLLPFSIHPGIPVSFFYRTEMNQNPSFNLSA KVWNSESPFRVGLKESNYQYAKYLGLFNEIKTQRKIIGKMNQLIA EDALLWQIAKKYLDKCHPKFSEIINQQVNGKQPLNVKAIHDTELTI DFPVDKNLYQVVVKMKQLDDFMFIDSKPVLEKTIRYMIKNSLRD KEYQQRVFIETNPGVFKIPYNEVVEAMRTINHQALTWARLLLDWE SKTVKTIPEADKKAKLQEAYNKRQPAFFNFNDICRYADDGKQLK DLRNKTFHTEVPETWTYDEMRENKTIKMVLGINETTFLKKDYAIE NARTIE |
| 22 | Cas13d_896 aa | MKKNSNDKTNAKRMGIKSFIKNGDERFITTSIKNEFPVELKLDVIK KTCEPAHEPVSFDYDPKKIDFEKPVLKEKLTSGQSGQKLSTRLFIQ KDRDICGIRRKYLEKIFNSNFIEEKKDSNLPMQIVAKVLSTEKVFSN ALNKIISQFLSMPRGGVTDNHGEYEIIGNIINHKSLQELNKEKKTKR IKKYLQSVIKNQSYLYNKQFLLSLDESKGSRNDIDENELYDYIRFL AILRNGIAHVFYEKNEPETAKESLFRLVDFIKNDKKLEGAFAKIKIQ VNTLYKCRKEEYIKKSGKNFEIIRKIYQNDKPDEKVKDWIRYDFD KSYKYIGLSVAKLGNYTSWAKDIDNLRDKSNPDSGYAGIMHRLN EFSVYLKVKALSTEEKDKYLKNLISKENCEEKDKYYKNIAQFFCSS DLKFANVLQMVKEIKKNKGCTSEDKNCKLCVDERKFNDLSVIVY FISCFLDNKDQNIFLSDLINRFGALSDLLRIQNKILGAGNKYNENYS FLKNERYVTEIKMELETIFALVKVSYKKEDKAFNRLLEDGLVMFG FSKDEAGMKVAGLKEIKEKKEGHYKNKSRSFLINSIVNSRKFAYL AKSIDPQKVPAIIKNEHIVRYILGRINKTNPGQIGRYWRYIMSQNH AGTDKVDDLTNEIIKINIKNILNDAGGWQKSKLNDNNNKKKLKYQ QLIGLYLTVAYIFVKNMLECNARYFSAFAQIEKDYLIYTNSDEFYY IDKNKKNLVTERYLKLVKDIIEKNKNTVRKDKIFRKKRQRKHLAD ISKSIIEFEKLPCCIFTLLRNITEHLNVASNIDIIEGYGKRAGKYHKN APASYFIFYHYIIQKILADKICTRNLLNIINTYGEPSISFIKIIYVPFAY NLPRYLNLTDARIFCNMDDK |
| 23 | Cas13d 911 aa | MQQNSTDNKNKIKKSAAKAVGVKSLARLSDGSTVFSSFGKGAAA |
| | | ELESLITGGEIRKLSDKAILEITDDTQNKNAYNVKSYRIPNLTARTD KLSDKSGMDDLGFKRELELEVFGQSFDDSIHIQIAHAVFDIQKSLA AVIPNVLYTLNNLDRSYSTDDTAGKKDIIGNTLNYQHSYDNFDKE KLGEFTEYYNAAKDRFSYFPDILCVLEKVNGKDRYQPKSEKDAFN VLSSVNMLRNSLFHFAQKSNDGKARIAVFKNQFDSDFSHITSTVN KIYSAKIAGVNENFLNNEGNNLYIILNATNWDIKKIVPQLYRFSVL KSDKNMGFNMRRLREFAVESKSIDLSRLNDKFLTNNRKKLYKVID FIIYYHLNKVRKDTFKDDFVAALRASQSEEEKEKLYAQYSERLFA DEGLKSAIKKAVDMISDTKSSIFKKKTPLDKALIENIKVNSDASDF CKLIYVFTRFLDGKEINILLNSLIKKFQDIHSFNTTVKKLSENNLIIN ADYVDDYSLFEQSGTVAKELMLIKSISKMDFGLDNINLSFMYDDA LRTLGVSDENLPEVKREYFGKTKNLSAYIRNNVLENRRFKYVIKYI HPSDVQKIACNKAIAGFVLNRMPDTQIKRYYDSLINKGATDMKA QAKALLDCITGISFDAIKDDKHLHKSKEKSPQRSADRERKKAMLT LYYTIVYIFVKQMLHINSLYTIGFFYLERDQRFIYSRAKKENKNSSK NSYLNDFRSVTAYFIPSEIMKRIEKNENKGFLEDFEALWNSCGKTS RLRKEDVLLYARYISPDHALKNYKMILNSYRNKIAHINVIMSAGK YTGGIKRMDSYFAVFQHLVQCDILSNPNNANCFKSESLKPLLLDM RFDGTDEKLYSKRLTRALNIPFGYNVPRYKNLTFEKIYLKSSINE |
| 24 | Cas13d_942 aa | MAKKKRITAKERKQNHRESLMKKADSNAEKEKAKKPVVENKPD TAISKDNTPKPNKEIKKSKAKLAGVKWVIKANDDVAYISSFGKGN NSVLEKRIMGDVSSNVNKDSHMYVNPKYTKKNYEIKNGFSSGSSL VTYPNKPDKNSGMDALCLKPYFEKDFFGHIFTDNMHIQAIYNIFDI EKILAKHITNIIYTVNSFDRNYNQSGNDTIGFDINYRVPYSEYGGG KDSNGEPKNQSKWKKRKNFIKFYNKSKPHLGYYENIFYDHGEPIS EEKFYNYLNILNFIRNNTFHYKDDDIELYSENYSEEFVFINCLNKFV KNKFKNVNKNFISNEKNNLYIILNAYGKDTENVEVVKKYSKELYK LSVLKTNKNLGVNVKKLRESAIEYGYCPLPYDKEKEVAKLSSVKH KLYKTYDFVITHYLNSNDKILLEIVEVLRLSKNDDEKENVYKKYA EKLFKADDVINPIKAISKLFAEKGNKLFKEKIIIKKEYIEDVSIDKNI YDFTKVIFFMTCFLDGKEINDLLTNIISKLQVIEDHNNVIKFISHNK DAVYKDYSDKYAIFRNAGKIATELEAIKSIARMENKIENAPQEPLL NDALLALGVSKTDLENTYNKYFDSKEKTDKQSQKVSTFLMNNVI NNNRFKYVIKYINPADINGLAKNRYLVKFVLSKIPEEQIDSYYKLF SNEEEPSCEEKIKLLTKKISKLNFQTLFENNKIPNVEKERKKAIITLY FTIVYILVKNLVNINGLYTLALYFVERDRYFYKKICGKALRRKVG DKYDYLLLPEIFSGSKYREETKNLKLPKEKDRDIMKKYLPNDKDR EGYNDFFTAYRNNIVHLNIIAKLSELTKNIDKDINSYFDIYHYCTQR VMFDYCKMNNNVVLAKMKDLAHIKSDCDEFSSKHTYPFSSAVLR FMNLPFAYNVPRFKNLSYKKFFDKQWLNH |
| 25 | Cas13d_957 aa | MAKKKRITAKERKQNHRESLMKKADSNAEKEKAKKPVVENKPD TAISKDNTPKPNKEIKKSKAKLAGVKWVIKANDDVAYISSFGKGN NSVLEKRIIGDVSSDVNKDSHMYVNPKYTKKNYEIKNGFSSGSSL TTHPNKPDKNSGMDALCLKIYFEKEIFKDKFNDNMHIQTIYNIFDI EKTLAKHITNIIYAVNSLDRSYIQSGNDTIGFGLNYRIPYAKYGRG KDSNGKPNNSNLKKRESFIKFYNNAKDRFGYFESVFYQNGKPISR EKLYIYLNILNFVRNSTFHYNNTSTYLYRKEYKYTDKDNCSVKEF EFVSYLNEFVKNKFKNVNKNFISNEKNNLYIILNAYGEDIEDVEVV KKYSKELYKLSVLKTNKNLGVNVKKLRESAIEYGYCPLPYDKEK EVAKLSSIKHKLYKTYDFVITHYLNSNDKLLLEIVEALRLSKNDDE KENVYKIYAEKIFKAEYVINPIKTISNLFAEKGDKLFNEKVSISEEY VEDIRIDKNIHNFTKVIFFLTCFLDGKEINDLLTNIISKLQVIEDHNN VIKAIANNNDAVYKDYSDKYAVFKNSGKIATKLEAIKSIARMENK INKAFKEPLLKDAMLALGVSPNDLDEKYEKYFKTDVDADKDHQK VSTFLMNNVINNSRFKYVVKYINPADINRLAKNKHLVKFVLDQIP HKQIDSYYNSVCTVEEPSYKGKIQLLTKKITGLNFYSLFENCKIPN VEKEKKKAVITLYFTIIYILVKNLVNINGLYTLALYFVERDGFFYK KICEKKDKKKTNKDVDYLLLPEIFSGSKYREETKNLKLPKEKDREI MKKYLPNDEDRKEYNKFFKQYRNNIVHLNIIANLSKLTSTIDKEIN SYFEIFHYCAQRVMFDYCKNNNKVVLAKMKDLAHIKSDCDEFSS KYTYPYSSAVLRFMNLPFAYNVPRFKNLSYQKFFDKQRLEALEKN LNI |
| 26 | Cas13d_968 aa | MGKKIHARDLREQRKNDRTTKFAEQNKKREAQMAVQKKDAAVS AKSVSSVSSKKGNVTKSMAKAAGVKSVFAVGKNTVYMTSFGRG NDAVLEQKIVDTSHEPLNIDDPAYQLNVVTMNGYSVTGHRGETV SAITDNPLRRFNGGKKDKPEQSVPADMLCLKPTLEKKFFGKEFDD NIHIQLIYNILDIEKILAVYSTNAVYALNNTIADENNENWDLFANFS TDNTYGELINAATYKESTDDVSTDDEKRREAEKKKREAKIAEKIL ADYEKFRKNNRLAYFADAFYIEKNKSKSKSQNKAEGIKRGKKEIY |
| | | SILALIAKLRHWCVHSEDGRAEFWLYKLDELEDDFKNVLDVVYN RPVEEINDDFVERNKVNIQILHSKCENSDIAELTRSYYEFLITKKYK NMGFSIKKLREIILEGTEYNDNKYDTVRNKLYQMVDFILYRGYIN ENSERAEALVNALRSTLNEDDKTKLYSSEAAFLKRKYMKIIREVT DSLDVKKLKELKKNAFTIPDNELRKCFISYADSVSEFTKLIYLLTRF LSGKEINDLVTTLINKFDNIRSFLEIMDELGLERTFTDEYSFFEGSTK YLAELIELNSFVKSCSFDMSAKRPMYRDALDILGIESDKSEDDIKR MIDNILQVDANGKKLPNKNHGLRNFIASNVVESNRFEYLVRYGNP KKIRETAKCKPAVRFVLNEIPDAQIERYYKAYYLDEKSLCLANMQ RDKLAGVIADIKFDDFSDAGSYQKANATSTKITSEAEIKRKNQAIIR LYLTVMYIMLKNLVNVNARYVIAFHCLERDTKLYAESGLEVGNIE KNKTNLTMAVMGVKLENGIIKTEFDKSLAENAANRYLRNARWY KLILDNLKMSERAVVNEFRNTVCHLNAIRNININIDGIKEVENYFA LYHYLIQKHLENRFADNGGSTGDYIGKLEEHKTYCKDFVKAYCTP FGYNLVRYKNLTIDGLFDKNYPGKDDSDKQK |
| 27 | Cas13d_982 aa | MGKKIHARDLREQRKNDRTTKFAEQNKKREAQMAVQKKDAAVS AKSVSSVSSKKGNVTKSMAKAAGVKSVFAVGKNTVYMTSFGRG NDAVLEQKIVDTSHEPLNIDDPAYQLNVVTMNGYSVTGHRGETV SAITDNPLRRFNGGKKDKPEQSVPADMLCLKPTLEKKFFGKEFDD NIHIQLIYNILDIEKILAVYSTNAVYALNNTIADENNENWDLFANFS TDNTYGELINAATYKESTDDVSTDDEKRREAEKKKREAKIAEKIL ADYEKFRKNNRLAYFADAFYIEKNKSKSKSQNKAEGIKRGKKEIY SILALIAKLRHWCVHSEDGRAEFWLYKLDELEDDFKNVLDVVYN RPVEEINDDFVERNKVNIQILHSKCENSDIAELTRSYYEFLITKKYK NMGFSIKKLREIILEGTEYNDNKYDTVRNKLYQMVDFILYRGYIN ENSERAEALVNALRSTLNEDDKTKLYSSEAAFLKRKYMKIIREVT DSLDVKKLKELKKNAFTIPDNELRKCFISYADSVSEFTKLIYLLTRF LSGKEINDLVTTLINKFDNIRSFLEIMDELGLERTFTDEYSFFEGSTK YLAELIELNSFVKSCSFDMSAKRPMYRDALDILGIESDKSEDDIKR MIDNILQVDANGKKLPNKNHGLRNFIASNVVESNRFEYLVRYGNP KKIRETAKCKPAVRFVLNEIPDAQIERYYKAYYLDEKSLCLANMQ RDKLAGVIADIKFDDFSDAGSYQKANATSTKITSEAEIKRKNQAIIR LYLTVMYIMLKNLVNVNARYVIAFHCLERDTKLYAESGLEVGNIE KNKTNLTMAVMGVKLENGIIKTEFDKSLAENAANRYLRNARWY KLILDNLKMSERAVVNEFRNTVCHLNAIRNININIDGIKEVENYFA LYHYLIQKHLENRFADNGGSTGDYIGKLEEHKTYCKDFVKAYCTP FGYNLVRYKNLTIDGLFDKNYPGKDDSDKQK |
| 28 | Cas13d_999 aa | MGNKQRVSAQKRRENAKLCNQQKARQAESQRDKIKNMNVEKM KNINTNDIKHTKTTAKKLGLKSTIIADKKIILTSFINEQSSKTANIEK VAGFKGDTIDTISYTPRMFRSEINPGEIVISKGDDLSEFANPANFPIG RDYVKIRSALEKQYFGKEFPEDNLHVQIAYNVADIKKILSVYINNII YMFYNLARSEEYDIFYNSQSENSGRDCDVIGSLYYQASYRNQDAN RFEKDGKKKAIDSLLDDTRAYYTYFDGLFSVPKREDDGKIKESEK EKAKDQNFDVLRLLSVGRQLTFHSDKSNNEAYLFDLSKLTRAAQ DENRRQDIQSLLNILNSTCRSNLEGVNGDFVKHAKNNLYVLNQLY PSLKANDLIGEYYNFIVKKENRNIGIRLITVRELIIEHNYTNLKDSK YDTYRNKIYTVLNFILFREIQENSIAIKNFREKLRSTEKAEQPALYQ AFANKIYPMVQAKFAKAIDLFEEQYKTKFKSEFKGGISIENMQQQ NILLQTENIDYFSKYVLFLTKFLDGKEINELLCALINKFDNIADLLDI SKQIGTPVVFCADYESLNDAAKIAENIRLIKNIAHLRPAIQEAQSSK DNADAAGTPATLLIDAYNMLNTDIQLVYGEAAYEELRKDLFERK NGTKYNKKGKKVDVYDHKFRNFLINNVIKSKWFFYIAKYVKPAD CAKMMSNKKMIEFALRDLPETQIKRYYYTITGNEALGDAESLKGV IIEQLHAFSIKNTLLSIKNMGEGEYKIQQIGSSKEKLKAIVNLYLTV AYLLTKSLVKVNIRFSIAFGCLERDLVLQKKSEKKFDAIINEILLED DKIRKECDKERAQAKTLPRELAQERFAQIKRRESGCYFKSYHVYD YLSKNSNEFKQNHIDFAVTSYRNNVEHLNVVHCMTKYFSEVKDV KSYYGVYCYIMQRMLCDELIIKNQDKPDVRQTFEEYNRLLKDHG TYSKNLMWLLNFPFAYNLARYKNLSNEDLFNAKNNDQKSK |

**Table 3:**

| SEQ ID NO. | crRNA name | Spacer sequence |
|---|---|---|
| 57 | mCherry-spacer-1 | GTTCATCACGCGCTCCCACTTGAAGCCCTC |
| 58 | mCherry-spacer-2 | TGCTTCACGTAGGCCTTGGAGCCGTACATG |
| 59 | NT-spacer-1 | CCTCTGAAACGATGGTGCATGGTAGTGACC |
| 60 | ANXA4-spacer-1 | AATTAGGCAGCCCTCATCAGTGCCGGCTCC |
| 61 | ANXA4-spacer-2 | CTTGTAGGCTGTCCTGATCTCCTGGCGCTG |
| 62 | EZH2-spacer_1 | GAGAGCAGCAGCAAACTCCTTTGCTCCCTC |
| 63 | EZH2-spacer_2 | CAGAGGAGCTCGAAGTTTCATCTTTCTTCT |
| 64 | EZH2-spacer_3 | GTATCCTTTGATTCCAGCACATTAATGGTG |
| 65 | NF2-spacer_1 | CTTGTGAACACTGGGGTCGTAGTCACCATA |
| 66 | NF2-spacer_2 | TAGTCACCATACTTGGCCTGGACGGCGTAA |
| 67 | NF2-spacer_3 | CCTTTTTGGAAGCAATTCCTCTTGGGCCAA |
| 68 | HRAS-spacer_1 | CTGTACTGGTGGATGTCCTCAAAAGACTTG |
| 69 | HRAS-spacer_2 | TCCGAGTCCTTCACCCGTTTGATCTGCTCC |
| 70 | HRAS-spacer_3 | TGTTCCCCACCAGCACCATGGGCACGTCAT |
| 71 | NRAS-spacer_1 | TACCAGTGTGTAAAAAGCATCTTCAACACC |
| 72 | NRAS-spacer_2 | CTGTAGAGGTTAATATCCGCAAATGACTTG |
| 73 | NRAS-spacer_3 | CGCTTAATCTGCTCCCTGTAGAGGTTAATA |
| 74 | PPARG-spacer_1 | CATTATGAGACATCCCCACTGCAAGGCATT |
| 75 | PPARG-spacer_2 | CGGCCTGTGGCATCCGCCCAAACCTGATGG |
| 76 | PPARG-spacer_3 | AAACCTGATGGCATTATGAGACATCCCCAC |
| 77 | STAT3-spacer | TAGCATCCATGATCTTATAGCCCATGATGA |
| 78 | EGFR-spacer | GTTTCTGGCAGTTCTCCTCTCCTGCACCCC |
| 79 | KRAS-spacer | GAAAGCCCTCCCCAGTCCTCATGTACTGGT |
| 80 | CXCR4-spacer | ATAAGGCCAACCATGATGTGCTGAAACTGG |

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13-2A-eGFP plasmid, 300ng pUC19-U6-5'-DR crRNA plasmid or pUC19-U6-3'-DR crRNA plasmid, and 10ng pCAG-eBFP-EF1a-mCherry plasmid. The control group was only transfected with 600ng pCAG-Cas13-2A-eGFP plasmid and 10ng pCAG-eBFP-EF1a-mCherry plasmid. After 48 hours, the cells were digested with 0.25% Trypsin-EDTA (Gibco), and the obtained suspension cells were subjected to fluorescence-activated cell sorting (FACS) (Fig. 11A).

### Targeting mCherry mRNA to identify novel Cas13 isoform crRNA directions

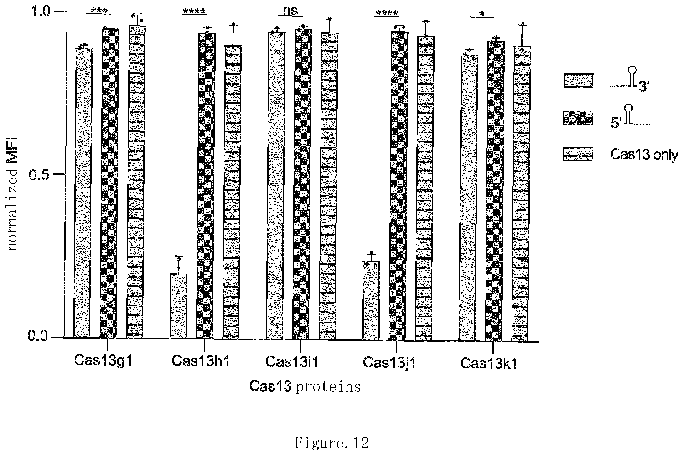

mCherry expression was analyzed by FACS 2 days after transfection. Comparing the expression of mCherry corresponding to 5'-DR crRNA and 3'-DR crRNA, it was found that the knockdown efficiency of 3'-DR crRNA corresponding to Cas13g1, Cas13h1, Cas13i1, Cas13j1, and Cas13k1 was higher than that of 5'-DR crRNA. It was proved that Cas13g-k corresponds to the crRNA direction of 3'-DR (Figure 12). The specific editing result information was shown in Table 4. In figure 12,each group had 3 biological replicates, and statistical significance was evaluated using a two-tailed t-test.

**Table 4:**

| | 3'-DR | 5'-DR |
|---|---|---|
| Cas13g1 | 0.889667 | 0.947 |
| Cas13h1 | 0.200667 | 0.938 |
| Cas13i1 | 0.943667 | 0.952333 |
| Cas13j1 | 0.244333 | 0.948333 |
| Cas13k1 | 0.878667 | 0.921667 |

### Example 4: Screening novel Cas13 proteins with high editing efficiency by targeting genes in vivo or in vitro

### Plasmid construction

The coding sequence of Cas13 was codon optimized (human) and synthesized. The synthesized Cas13 effector protein sequence was inserted into the XmaI and NheI restriction sites of the pCAG-2A-eGFP vector to construct the pCAG-Cas13-2A-eGFP plasmid. The protein sequences used were shown in Table 2. The synthesized crRNA sequence (U6 promoter sequence + spacer sequence + DR sequence) was inserted between the EcoRI and HindIII restriction sites of the pUC19-U6 vector to construct the pUC19-U6-3'-DR crRNA plasmid. The spacer sequence information was shown in Table 3. The CAG promoter expressed eBFP protein and the EF1a promoter expressed mCherry protein to construct pCAG-eBFP-EF1a-mCherry plasmid (Figure 11A).

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13-2A-eGFP plasmid, 300ng pUC19-U6-3'-DR crRNA plasmid, and 10ng pCAG-eBFP-EF1a-mCherry plasmid. The control group was only transfected with 600ng pCAG-Cas13-2A-eGFP plasmid and 10ng pCAG-eBFP-EF1a-mCherry plasmid. 600ng pCAG-Cas13-2A-eGFP plasmid, 300ng non-targeting (NT) U6-crRNA plasmid and 10ng pCAG-eBFP-EF1a-mCherry plasmid were co-transfected into HEK293T cells as a non-targeting control group. After 48 hours, the cells were digested with 0.25% Trypsin-EDTA (Gibco), and the obtained suspension cells were subjected to fluorescence-activated cell sorting (FACS) (Fig. 11A).

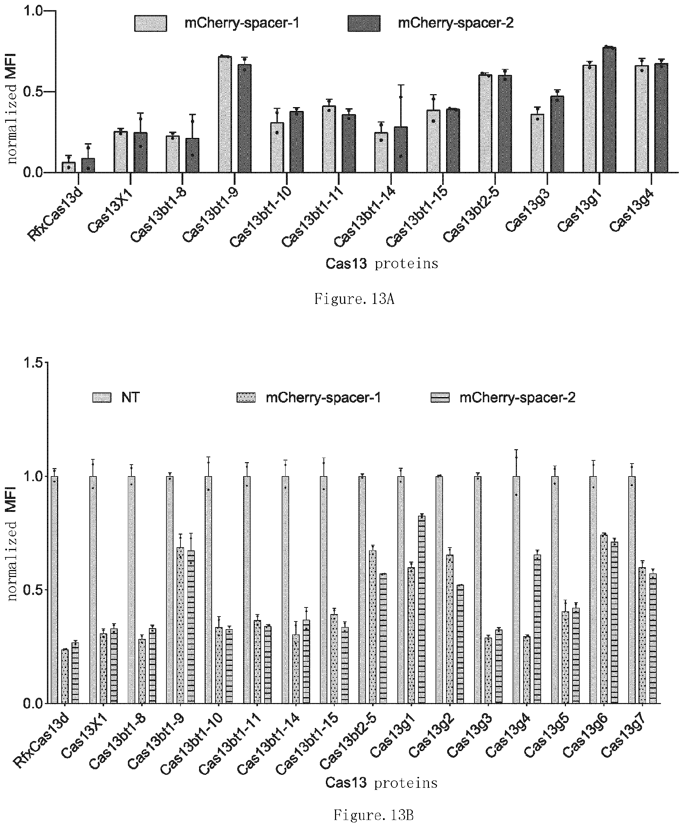

Knocking down mCherry mRNA to screen for Cas13 proteins with high editing efficiency mCherry expression was analyzed by flow cytometry 2 days after transfection. The mean fluorescence intensity (MFI) of EGFP and mCherry double-positive cells in the experimental group was normalized to that of the control group. Preliminary screening of Cas13 proteins with high editing efficiency (Figure 13A) was performed, wherein the knockdown efficiencies of RfxCas13d, Cas13X1, Cas13bt1-8, Cas13bt1-10, Cas13bt1-11, Cas13bt1-14, Cas13bt1-15, and Cas13g3 were higher than 50%. These proteins were used for the following experiments, the specific editing result information was shown in Table 5A.

mCherry expression was analyzed by flow cytometry 2 days after transfection. The MFI of EGFP and mCherry double-positive cells in the experimental group was normalized to that of the non-target control group. The MFI results (Figure 13B) and specific editing result information were shown in Table 5B.

**Table 5A:**

| | mCherry-spacer-1 | mCherry-spacer-2 |
|---|---|---|
| RfxCas 13 d | 0.06285 | 0.08915 |
| Cas13X1 | 0.2535 | 0.2475 |
| Cas13bt1-8 | 0.227 | 0.2125 |
| Cas13bt1-9 | 0.718 | 0.6685 |
| Cas13bt1-10 | 0.3095 | 0.3785 |
| Cas13bt1-11 | 0.413 | 0.359 |
| Cas13bt1-14 | 0.2475 | 0.28335 |
| Cas13bt1-15 | 0.387 | 0.3925 |
| Cas13bt2-5 | 0.606 | 0.6025 |
| Cas13g3 | 0.3625 | 0.474 |
| Cas13g1 | 0.6665 | 0.7745 |
| Cas13g4 | 0.6625 | 0.675 |

**Table 5B:**

| | mCherry-spacer-1 | mCherry-spacer-2 |
|---|---|---|
| RfxCas 13 d | 0.2372055 | 0.2672625 |
| Cas13X1 | 0.307399 | 0.3295195 |
| Cas13bt1-8 | 0.2822405 | 0.33014 |
| Cas13bt1-9 | 0.68599 | 0.6714975 |
| Cas13bt1-10 | 0.333556 | 0.326203 |
| Cas13bt1-11 | 0.3649895 | 0.3401845 |
| Cas13bt1-14 | 0.303531 | 0.3666415 |
| Cas13bt1-15 | 0.391273 | 0.3352725 |
| Cas13bt2-5 | 0.6732165 | 0.5691415 |
| Cas13g1 | 0.5981245 | 0.8246755 |
| Cas13g2 | 0.653143 | 0.5203655 |
| Cas13g3 | 0.2890905 | 0.324154 |
| Cas13g4 | 0.294376 | 0.653641 |
| Cas13g5 | 0.4043715 | 0.4201575 |
| Cas13g6 | 0.7418645 | 0.7109505 |
| Cas13g7 | 0.597749 | 0.5702335 |

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13-2A-eGFP plasmid and 300ng pUC19-U6-3'-DR crRNA plasmid. The control group was only transfected with 600ng pCAG-Cas13-2A-eGFP plasmid (Figure 11B). The non-target control group was transfected with 600ng pCAG-Cas13-2A-eGFP plasmid and 300ng non-target U6-crRNA plasmid.

### RNA extraction, RNA quantitative analysis

48 hours after transfection, cells were lysed using TRIzol^{®} Reagent(Life Technologie), and then RNA was extracted using PureLink^{™} RNA Mini Kit (Thermofisher). Finally, RNA quantitative analysis was performed using HiScript^{®} II One Step qRT-PCR SYBR Green Kit(Vazyme). RT-qPCR results were analyzed using the 2^{-ΔΔCT} method. The difference in average CT values between the target gene and the internal reference gene *GAPDH* in three biological replicates was used to calculate the relative expression of the target gene, and the relative expression of the control group was normalized.

### Knocking down the gene ANXA4 mRNA in vivo to screen for Cas13 proteins with high editing efficiency

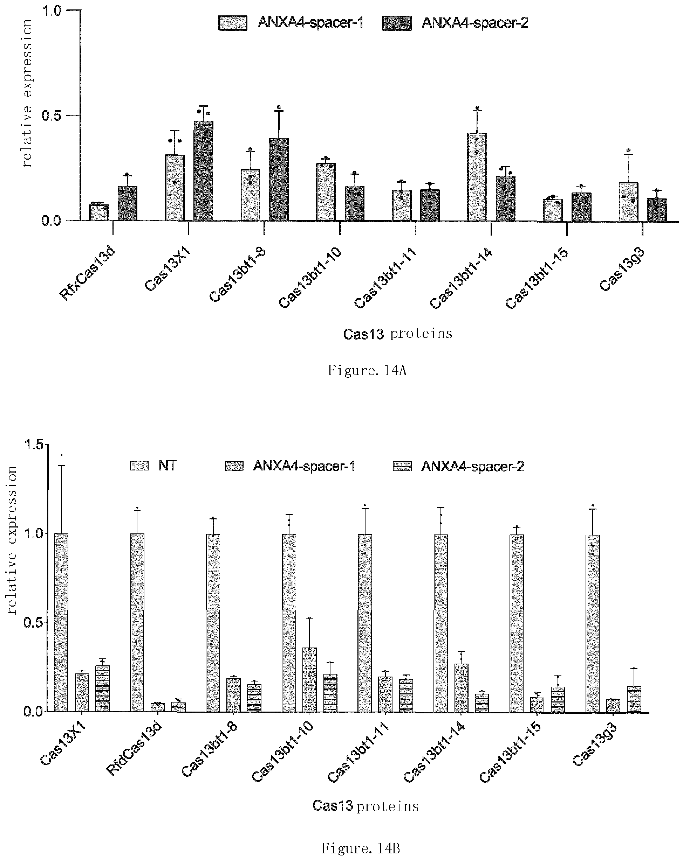

Through the normalized RT-qPCR results targeting ANXA4 mRNA and the control group, it was found that Cas13bt1-11, Cas13bt1-15 and Cas13g3 have higher editing efficiency (Figure 14A). The specific editing result information was shown in Table 6A. The normalized RT-qPCR results of the experimental group and the non-target control group also showed that Cas13bt1-8, Cas13bt1-11, Cas13bt1-15 and Cas13g3 had higher editing (Figure 14B). The specific editing result information was shown in Table 6B. It was worth noting that both experiments showed that Cas13g3 has the strongest RNA interference ability and an ultra-small protein molecular weight (767 amino acids). We selected the Cas13g3 protein for further characterization analysis.

**Table 6A:**

| | ANXA4-spacer-1 | ANXA4-spacer-2 |
|---|---|---|
| RfxCas 13 d | 0.073333 | 0.163333 |
| Cas13X1 | 0.313333 | 0.473333 |
| Cas13bt1-8 | 0.243333 | 0.393333 |
| Cas13bt1-10 | 0.273333 | 0.166667 |
| Cas13bt1-11 | 0.146667 | 0.15 |
| Cas13bt1-14 | 0.42 | 0.213333 |
| Cas13bt1-15 | 0.106667 | 0.136667 |
| Cas13g3 | 0.186667 | 0.11 |

**Table 6B:**

| | ANXA4-spacer-1 | ANXA4-spacer-2 |
|---|---|---|
| RfxCas13d | 0.215066 | 0.258412 |
| Cas13X1 | 0.046716667 | 0.054699333 |
| Cas13bt1-8 | 0.188513333 | 0.153782667 |
| Cas13bt1-10 | 0.363544667 | 0.214909 |
| Cas13bt1-11 | 0.200584333 | 0.188127667 |
| Cas13bt1-14 | 0.275353333 | 0.105536 |
| Cas13bt1-15 | 0.086085667 | 0.146604667 |
| Cas13g3 | 0.076056 | 0.150550667 |

### Knockdown of genes in vivo to screen for Cas13 proteins with high editing efficiency

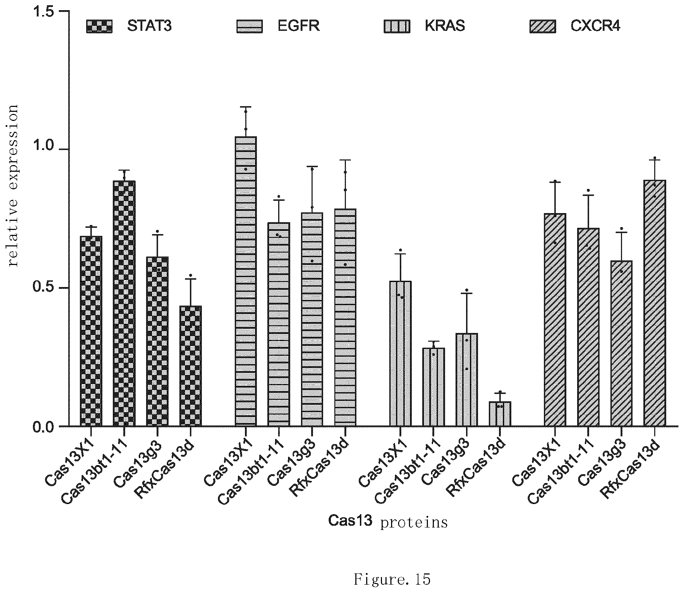

By targeting 4 in vivo genes, it was found that the editing efficiency of RfxCas13d at *STAT3* and *KRAS* sites was higher than that of Cas13g3, while the editing efficiency at EGFR and CXCR4 was lower. Overall, the editing efficiency of Cas13g3 was slightly lower than that of RfxCas13d, but exceeded that of Cas13X1 (Figure 15), the editing results were shown in Table 7.

**Table 7:**

| | STAT3-spacer | EGFR-spacer | KRAS-spacer | CXCR4-spacer |
|---|---|---|---|---|
| Cas13X1 | 0.687478 | 1.046535 | 0.525891 | 0.769662 |
| Cas13bt1-11 | 0.886987 | 0.735842 | 0.284075 | 0.71624 |
| Cas13g3 | 0.613047 | 0.772218 | 0.336777 | 0.598634 |
| RfxCas13d | 0.435223 | 0.785867 | 0.08919 | 0.890071 |

### Example 5: Verification of Cas13d homolog editing efficiency by targeting genes in vivo Plasmid construction

The coding sequence of the Cas13d homolog was codon optimized (human) and synthesized. The synthesized Cas13 effector protein sequence was inserted into the XmaI and NheI restriction sites of the pCAG-2A-eGFP vector to construct the pCAG-Cas13d-2A-eGFP plasmid. The protein sequences used were shown in Table 2. The synthesized crRNA sequence (U6 promoter sequence + DR sequence + spacer sequence) was inserted between the EcoRI and HindIII restriction sites of the pUC19-U6 vector to construct the pUC19-U6-5'-DR crRNA plasmid. The spacer sequence information was shown in Table 3.

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13d-2A-eGFP plasmid and 300ng pUC19-U6-5'-DR crRNA plasmid. The control group was only transfected with 600ng pCAG-Cas13d-2A-eGFP plasmid (Figure 11B).

### RNA extraction, RNA quantitative analysis

48 hours after transfection, cells were lysed using TRIzol^{®} reagent(Life Technologie), and then RNA was extracted using PureLink^{™} RNA Mini Kit (Thermofisher). Finally, RNA quantitative analysis was performed using HiScript^{®} II One Step qRT-PCR SYBR Green kit(Vazyme). RT- qPCR results were analyzed using the 2^{-ΔΔCT} method. The difference in average CT values between the target gene and the internal reference gene *GAPDH* in three biological replicates was used to calculate the relative expression of the target gene, and the relative expression of the control group was normalized.

### Knocking down the gene ANXA4 mRNA in vivo to screen for Cas13 proteins with high editing efficiency

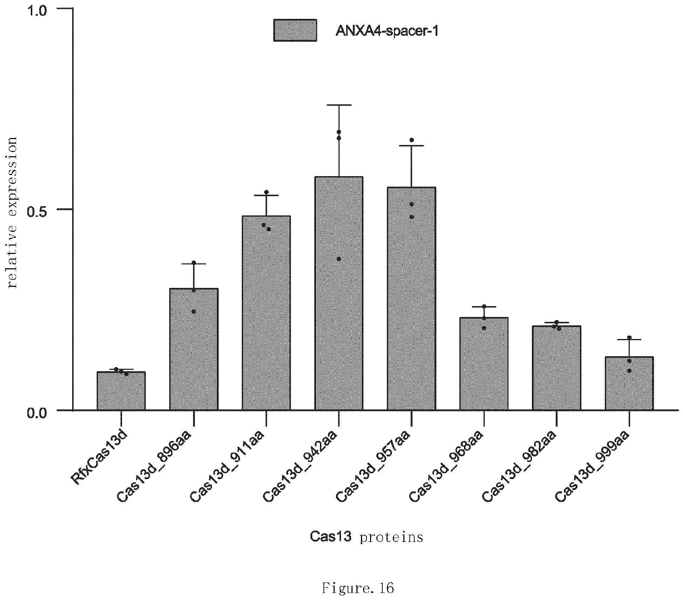

Through the RT-qPCR results targeting ANXA4 mRNA, it was found that Cas13d_968aa, Cas13d_982aa and Cas13d_999aa have high editing efficiency (Figure 16). The specific editing result information was shown in Table 8.

**Table 8:**

| | ANXA4-spacer-1 |
|---|---|
| RfxCas 13 d | 0.096407 |
| Cas13d_896aa | 0.303705 |
| Cas13d_911aa | 0.484585 |
| Cas13d_942aa | 0.582031 |
| Cas13d_957aa | 0.555765 |
| Cas13d_968aa | 0.230899 |
| Cas13d_982aa | 0.210199 |
| Cas13d_999aa | 0.13415 |

### Example 6: Using RNA-seq to explore the off-target activity of Cas13

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13-2A-eGFP plasmid and 300ng pUC19-U6-3'-DR crRNA plasmid, with crRNA targeting ANXA4 mRNA. The control group was only transfected with 600ng pCAG-Cas13-2A-eGFP plasmid.

### RNA-seq

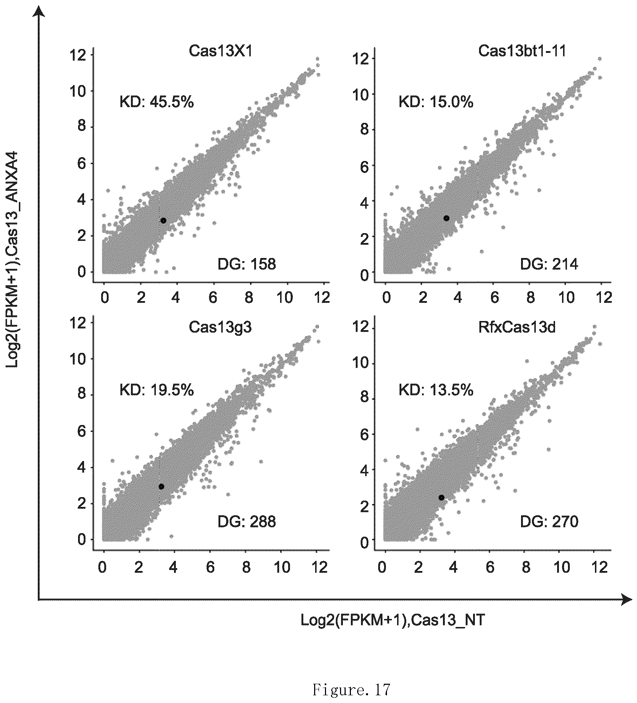

Two days after transfection, cells were lysed using TRIzol^{®} reagent(Life Technologie), and then RNA was extracted using PureLinkTM RNA Mini Kit (Thermofisher). Total RNA was extracted and RNA-seq sequenced using the novaseq 6000 platform. The obtained sequencing results were compared to the human genome through STAR software, and then gene expression was calculated through StringTie software. Compared with the control group, it was found that the off-target sites corresponding to Cas13X1, Cas13g3 and RfxCas13d were 102, 133 and 323 respectively. It showed that the specificity of Cas13g3 was comparable to Cas13X1, while its off-target effect was lower than that of RfxCas13d (Figure 17). In Figure 17, KD represented the knockdown efficiency, and DG represented the number of down-regulated genes.

### Example 7: Exploring the trans-cleavage activity of Cas13

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection.

### Cell viability analysis

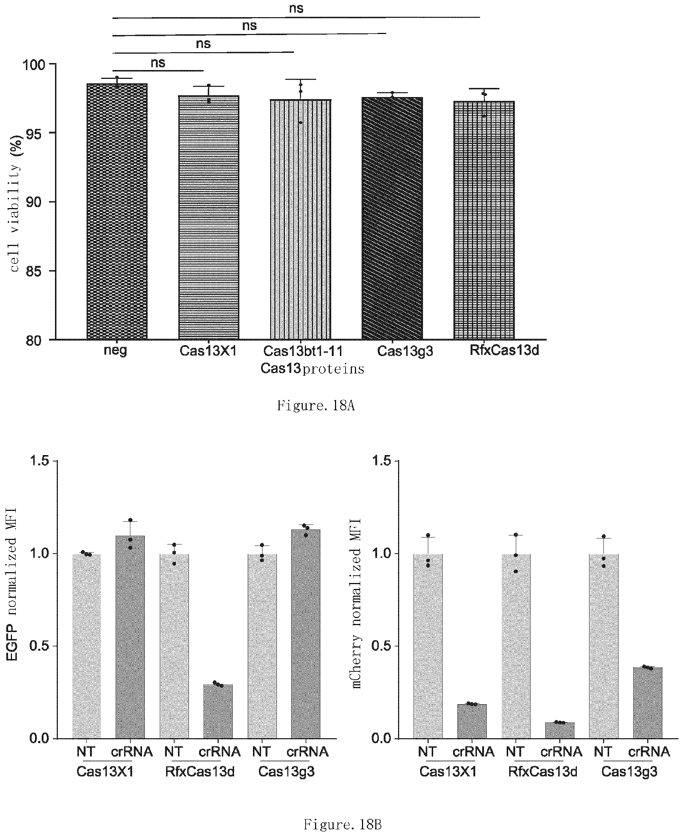

Each well of the cell viability experimental group was transfected with 600ng pCAG-Cas13-2A-eGFP plasmid and 300ng pUC19-U6-3'-DR crRNA plasmid, with crRNA targeting ANXA4 mRNA. The control group was not transfected with any plasmid. Calcein-AM/PI double strain kit (Solarbio) was used to measure fluorescent cell viability of HEK293T 2 days after transfection. Total cell quantification was performed at 490nm excitation and 515nm emission, and dead cell quantification was performed at 535nm excitation and 617nm emission. Measurements were made using flow cytometry. By identifying the viability of transfected cells, it was found that transfection with Cas13 protein did not affect cell viability (Figure 18A). The specific cell viability information is shown in Table 9A. In Figure 18, there were 3 biological replicates for each group, and statistical significance was evaluated using a two-tailed t-test.

**Table 9A:**

| Neg | Cas13X1 | Cas13bt1-11 | Cas13g3 | RfxCas13d |
|---|---|---|---|---|
| 98.56 | 97.68667 | 97.40333 | 97.57333 | 97.28 |

### Trans-cleaving activity

Each well of the trans-cleaving experimental group was transfected with 600ng pCAG-Cas13-2A-eGFP plasmid, 10ng pCAG-eBFP-EF1a-mCherry plasmid and 300ng pUC19-U6-3'-DR crRNA plasmid, in which crRNA targeted mCherry mRNA. The trans-cleaving control group was transfected with non-targeting U6-crRNA plasmid. The cis-cleaving ability of Cas13 protein was calculated by measuring the fluorescence intensity of mCherry, and the trans-cleaving ability was calculated by measuring the fluorescence intensity of EGFP. Consistent with previous results, Cas13X1, RfxCas13d and Cas13g3 could significantly knock down mCherry mRNA (Figure 18B). Meanwhile, no trans-cleaving activity was detected for Cas13X.1 and Cas13g3, while RfxCas13d showed significant trans-cleaving activity against EGFP transcript (Fig. 18B). The specific MFI result information was seen in Table 9B.

**Table 9B:**

| | GFP MFI | mCherry MFI |
|---|---|---|
| Cas13X1 | 1.096344 | 0.188002667 |
| RfdCas13d | 0.294856 | 0.088376333 |
| Cas13g3 | 1.130295 | 0.385392333 |

### Example 8: Characterization of Cas13g3 system with high editing efficiency

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection.

### Research on the optimal spacer length of Cas13g3 system

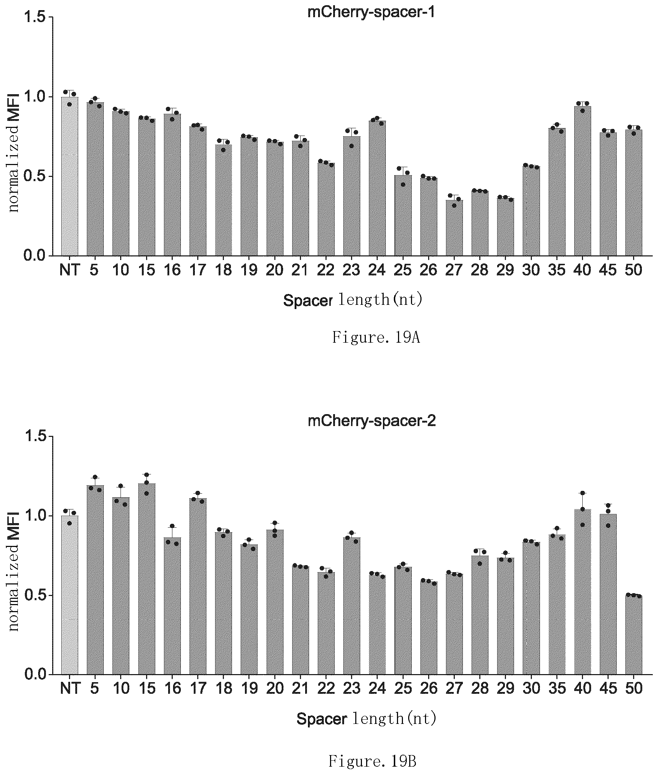

In order to explore the optimal spacer length of the Cas13g3 system, we constructed U6-crRNA plasmids targeting mCherry mRNA with spacer lengths ranging from 5 to 50 nt. When the spacer length is between 15-30nt, a crRNA was designed every 1 nt; when the spacer length is 5-15nt and 30-50nt, a crRNA was designed every 5nt. 600ng pCAG-Cas13-2A-eGFP plasmid, 10ng pCAG-eBFP-EF1a-mCherry plasmid and 300ng pUC19-U6-3'-DR crRNA plasmid were co-transfected into HEK293T cells as the experimental group. 600ng pCAG-Cas13-2A-eGFP plasmid, 300ng non-targeting U6-crRNA plasmid and 10ng pCAG-eBFP-EF1a-mCherry plasmid were co-transfected into HEK293T cells as a control group. The MFI of EGFP and mCherry double-positive cells in the experimental group was normalized with the negative control. The normalized results can reflect the impact of different spacer lengths on RNA interference. The results showed that when using a spacer with a length of 27nt, Cas13g3 had the highest average knockout efficiency at the two target sites (Figure 19). The specific editing result information was seen in Table 10.

**Table 10:**

| mCherry-spacer-1(nt) | normalized MFI | mCherry-spacer-2(nt) | normalized MFI |
|---|---|---|---|
| 5 nt | 0.966452 | 5 nt | 1.192277 |
| 10 nt | 0.908946 | 10 nt | 1.116929667 |
| 15 nt | 0.862876333 | 15 nt | 1.202048333 |
| 16 nt | 0.894542333 | 16 nt | 0.864939333 |
| 17 nt | 0.813911333 | 17 nt | 1.111863333 |
| 18 nt | 0.702482667 | 18 nt | 0.896605333 |
| 19 nt | 0.745549 | 19 nt | 0.820027667 |
| 20 nt | 0.717067333 | 20 nt | 0.912239333 |
| 21 nt | 0.724233 | 21 nt | 0.68218 |
| 22 nt | 0.583201 | 22 nt | 0.646931 |
| 23 nt | 0.752605667 | 23 nt | 0.864504667 |
| 24 nt | 0.850934 | 24 nt | 0.630356 |
| 25 nt | 0.506514333 | 25 nt | 0.678995333 |
| 26 nt | 0.490626667 | 26 nt | 0.586783667 |
| 27 nt | 0.35021 | 27 nt | 0.636363667 |
| 28 nt | 0.407426333 | 28 nt | 0.750615333 |
| 29 nt | 0.363202 | 29 nt | 0.738346667 |
| 30 nt | 0.562174333 | 30 nt | 0.835335667 |
| 35 nt | 0.805189667 | 35 nt | 0.88488 |
| 40 nt | 0.943326667 | 40 nt | 1.042414667 |
| 45 nt | 0.777468333 | 45 nt | 1.010639667 |
| 50 nt | 0.795888667 | 50 nt | 0.500325667 |

### Cas13g3 system PFS preference analysis

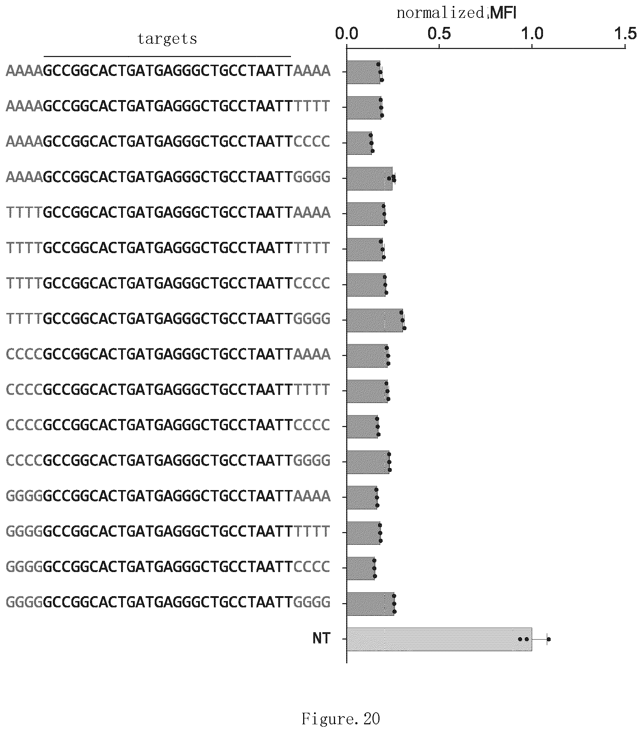

To analyze the PFS preference of the Cas13g3 system, targets of 16 PFS combinations were cloned upstream of the mCherry gene of the EF-1α-mCherry plasmid. The MFI of EGFP and mCherry double-positive cells in the experimental group was normalized with the MFI of the negative control. The experimental group was HEK293T cells transfected with 600ng pCAG-Cas13-2A-eGFP plasmid, 10ng pCAG-eBFP-EF1a-mCherry plasmid and 300ng pUC19-U6-3'-DR crRNA plasmid; the negative control was HEK293T cells transfected with 600ng pCAG-Cas13-2A-eGFP plasmid, 300ng non-targeting U6-crRNA plasmid and 10ng pCAG-eBFP-EF1a-mCherry plasmid. The results showed that Cas13g3 protein demonstrated stable and efficient interference effects in these 16 PFS sequences (Figure 20). The specific editing result information was shown in Table 11 (the spacer sequence was: SEQ ID NO. 81: GCCGGCACTGATGAGGGCTGCCTAATT).

**Table 11:**

| PFS combination | normalized MFI |
|---|---|
| AAAA-spacer-AAAA | 0.18097067 |
| AAAA-spacer-TTTT | 0.18666533 |
| AAAA-spacer-CCCC | 0.134758 |
| AAAA-spacer-GGGG | 0.24784567 |
| TTTT-spacer-AAAA | 0.20475733 |
| TTTT-spacer-TTTT | 0.19346833 |
| TTTT-spacer-CCCC | 0.21004867 |
| TTTT-spacer-GGGG | 0.303835 |
| CCCC-spacer-AAAA | 0.22320233 |
| CCCC-spacer-TTTT | 0.22158933 |
| CCCC-spacer-CCCC | 0.16811967 |
| CCCC-spacer-GGGG | 0.23202133 |
| GGGG-spacer-AAAA | 0.16308 |
| GGGG-spacer-TTTT | 0.18122267 |
| GGGG-spacer-CCCC | 0.15048133 |
| GGGG-spacer-GGGG | 0.258429 |

### Effect of NLS on the interference activity of Cas13g3 system

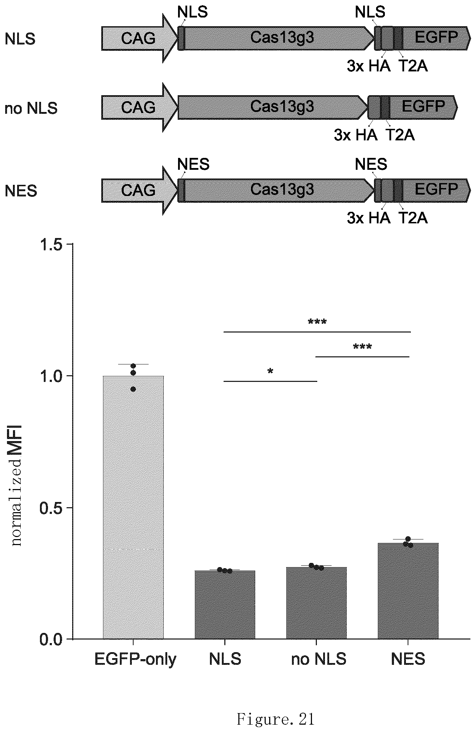

In order to identify the effects of NLS and NES on the interference activity of the Cas13g3 system, we constructed three Cas13 protein expression plasmids fused with NLS, fused with NES, and without fused NLS and NES respectively (Figure 21). 600ng pCAG-Cas13-2A-eGFP plasmid, 10ng pCAG-eBFP-EF1a-mCherry plasmid and 300ng pUC19-U6-3'-DR crRNA plasmid were co-transfected into HEK293T cells as the experimental group. The control group was only transfected with pCAG-eGFP plasmid. The MFI of EGFP and mCherry double-positive cells was analyzed and calculated. The normalized results compared with the control group showed that the fusion of NLS sequences can improve the knockout efficiency of Cas13g3 compared to the fusion of NES or the absence of any localization signal (Figure 21). The specific editing result information was seen in Table 12.

**Table 12:**

| | normalized MFI |
|---|---|
| NLS | 0.260501 |
| noNLS | 0.274187 |
| NES | 0.367439 |

Therefore, the optimal Cas13g3 editor composition is a crRNA plasmid with a spacer length of 27nt and a Cas13g3 protein plasmid fused with the NLS sequence.

### Example 9: Optimal Cas13g3 editor interference capability verification

### Cell culture, transfection

HEK293T cells were cultured in DMEM (Gibco) medium containing 10% FBS (Gibco) and 1% Penicillin-Streptomycin (Gibco). The cells were digested with 0.25% Trypsin-EDTA (Gibco), transferred to a 24-well plate and cultured for 12 hours. When the cell density reached 90%, LIPOFECTAMINE 3000 Reagent (Invitrogen) was used for transfection. Each well of the experimental group was transfected with 600ng pCAG-Cas13-2A-eGFP plasmid and 300ng pUC19-U6-3'-DR crRNA plasmid. The non-target control group was transfected with 600ng pCAG-Cas13-2A-eGFP plasmid and 300ng non-target U6-crRNA plasmid.

### RNA extraction, RNA quantitative analysis

48 hours after transfection, cells were lysed using TRIzol^{®} Reagent(Life Technologie), and then RNA was extracted using PureLinkTM RNA Mini Kit (Thermofisher). Finally, RNA quantitative analysis was performed using HiScript^{®} II One Step qRT-PCR SYBR Green Kit(Vazyme). RT-qPCR results were analyzed using the 2^{-ΔΔCT} method. The difference in average CT values between the target gene and the internal reference gene *GAPDH* in three biological replicates was used to calculate the relative expression of the target gene, and the relative expression of the control group was normalized.

### Comparison of RNA interference capabilities between the Cas13g3 system and other Cas13 systems

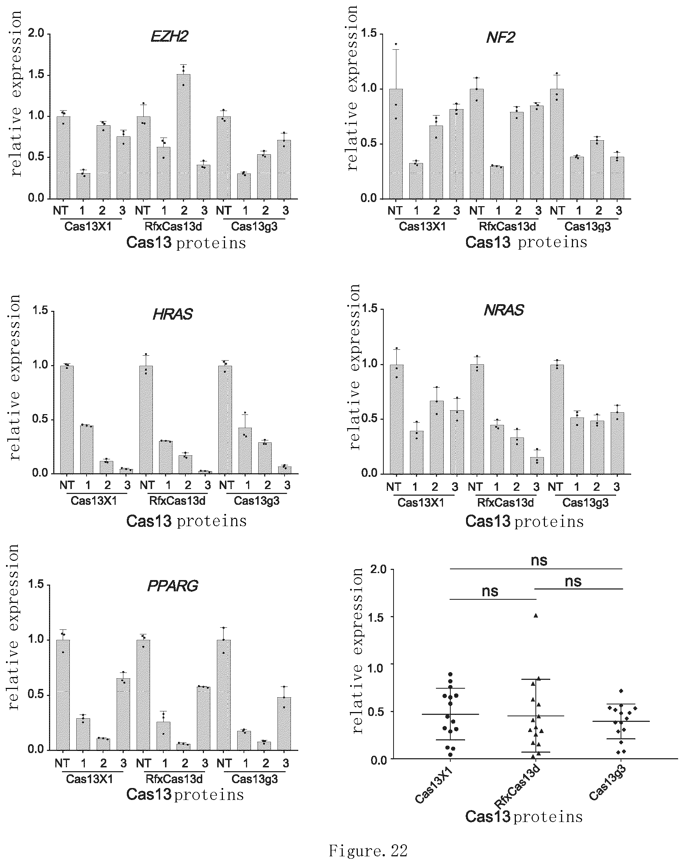

In order to systematically compare the RNA interference activity of Cas13g3 protein with the currently most commonly used and highest cleavage efficiency Cas13 proteins Cas13X1 and RfxCas13d at the same target site and study the knockout efficiency of Cas13g3 protein on a larger scale, we designed a total of 15 crRNAs plasmids targeting 5 endogenous genes. By targeting 5 in vivo genes *EZH2, NF2, HRAS, NRAS* and *PPARG,* Cas13X1, RfxCas13d and Cas13g3 can exhibit powerful RNA interference activity on *EZH2, NF2, HRAS, NRAS* and *PPARG* genes, and their average knockdown efficiencies are respectively 52.84%, 54.58% and 60.37% (Figure 22). The specific editing results were shown in Table 13. Paired-t-test statistical analysis of the interference results showed that Cas13g3 has efficient RNA knockdown activity, which is comparable to Cas13X1 and RfxCas13d.

**Table 13:**

| | Cas13X1 | RfdCas13d | Cas13g3 |
|---|---|---|---|
| EZH2-spacer-1 | 0.313798 | 0.627438 | 0.306951 |
| EZH2-spacer-2 | 0.755891 | 0.408723 | 0.715392 |
| EZH2-spacer-3 | 0.89017 | 1.51347 | 0.539906 |
| NF2-spacer-1 | 0.324943 | 0.296356 | 0.381387 |
| NF2-spacer-2 | 0.666154 | 0.791354 | 0.533823 |
| NF2-spacer-3 | 0.817664 | 0.847641 | 0.383713 |
| HRAS-spacer-1 | 0.044778 | 0.024482 | 0.06682 |
| HRAS-spacer-2 | 0.446724 | 0.304201 | 0.425856 |
| HRAS-spacer-3 | 0.119186 | 0.169341 | 0.289796 |
| NRAS-spacer-1 | 0.393875 | 0.449508 | 0.51671 |
| NRAS-spacer-2 | 0.668483 | 0.333773 | 0.485217 |
| NRAS-spacer-3 | 0.584562 | 0.154314 | 0.564822 |
| PPARG-spacer-1 | 0.288989 | 0.259088 | 0.174556 |
| PPARG-spacer-2 | 0.107358 | 0.059001 | 0.077466 |
| PPARG-spacer-3 | 0.651076 | 0.57364 | 0.481814 |

Although the embodiments of the present invention have been described above in conjunction with the accompanying drawings, the present invention is not limited to the above-mentioned specific embodiments and application fields. The above-mentioned specific embodiments are only illustrative and instructive, rather than restrictive. Under the inspiration of this description and without departing from the scope of protection of the claims of the present invention, those of ordinary skill in the art can also make many forms, which are all included in the protection of the present invention.

## Claims

1. An isolated Cas13 nuclease protein, the amino acid sequence of the Cas13 nuclease protein is:
a) the amino acid sequence shown in any one of SEQ ID NOs. 1 to 28; or
b) an amino acid sequence having at least 80% sequence identity with any one of SEQ ID NOs. 1 to 28 and having RNA cleavage activity.

2. The Cas13 nuclease protein of claim 1, wherein the Cas13 nuclease protein is Cas13bt1, Cas13bt2, Cas13g, Cas13h, Cas13i, Cas13j or Cas13k protein, preferably Cas13g3.

3. An engineered Cas13 nuclease effector protein, comprising a Cas13 nuclease protein, the Cas13 nuclease protein comprising:
a) the amino acid sequence shown in any one of SEQ ID NOs. 1 to 28; or,
b) an amino acid sequence having at least 80% sequence identity with any one of SEQ ID NOs. 1 to 28 and having RNA cleavage activity.

4. The effector protein of claim 3, wherein the Cas13 nuclease protein loses its catalytic activity through amino acid mutation, for example, the Cas13 nuclease protein forms dCas13 protein through amino acid mutations in the HEPN domain (RxxxxH motif) at its C-terminus and/or N-terminus.

5. The effector protein of claim 3 or 4, further comprising a functional domain fused to the Cas13 nuclease protein.

6. The effector protein of claim 5, wherein the functional domain is selected from one or more of the following: translation initiation domain, translation repression domain, transactivation domain, epigenetic modification domain, nucleobase editing domain, reverse transcriptase domain, reporter domain and nuclease domain.

7. The effector protein of claim 6, wherein the nucleobase editing domain is adenosine deaminase, cytidine deaminase or their catalytic domains.

8. A polynucleotide, encoding the Cas13 nuclease protein of claim 1 or 2 or the effector protein of any one of claims 3-7.

9. A vector, comprising the polynucleotide of claim 8, wherein the vector is a plasmid or a virus, and the virus is preferably a lentivirus.

10. An engineered CRISPR-Cas13 gene editing system, comprising:
(a) the nuclease of claim 1 or 2, the engineered Cas13 nuclease effector protein of any one of claims 3-7, or the nucleic acid encoding the effector protein; and
(b) crRNA, comprising a spacer sequence complementary to a target sequence in a target nucleic acid;
wherein, the engineered Cas13 nuclease effector protein and the crRNA can form a CRISPR complex that specifically binds to a target nucleic acid comprising the target sequence and induces modification of the target nucleic acid.

11. The engineered CRISPR-Cas13 gene editing system of claim 10, wherein the crRNA further comprises a direct repeat (DR) sequence, preferably the direct repeat (DR) sequence comprises any one of SEQ ID NOs. 29 to 56 or a sequence having at least 80% identity with the sequence shown in any one of SEQ ID NOs. 29 to 56.

12. A kit, comprising the engineered CRISPR-Cas13 gene editing system of claim 10 or 11.

13. Use of the Cas13 nuclease protein of claim 1 or 2, the engineered CRISPR-Cas13 nuclease effector protein of any one of claims 3-7, the engineered CRISPR-Cas13 gene editing system of claim 10 or 11 in the preparation of a medicament for the treatment of diseases or conditions associated with nucleic acid mutations in cells of an individual.

14. A method for modifying a cell comprising a target nucleic acid, comprising contacting the cell with the Cas13 nuclease protein of claim 1 or 2, the engineered Cas13 nuclease effector protein of any one of claims 3-7, the engineered CRISPR-Cas13 gene editing system of claim 10 or 11, thereby achieving modification of the target nucleic acid in the cell.

15. A composition comprising the Cas13 nuclease protein of claim 1 or 2, the engineered Cas13 nuclease effector protein of any one of claims 3-7, the engineered CRISPR-Cas13 gene editing system of claim 10 or 11, for modification of the nucleic acids.
